# EUROPEAN PATENT SPECIFICATION

(11) **EP 4 042 160 B1**
(45) Date of publication and mention of the grant of the patent: **27.03.2024**
(21) Application number: 20793828.3
(22) Date of filing: 11.10.2020
(51) Int. Cl.: G01N 33/574

(54) **USE OF MECHANICAL PROPERTIES OF CELLS IN CANCER DETECTION METHODS**
VERWENDUNG MECHANISCHER EIGENSCHAFTEN VON ZELLEN IN METHODEN ZUR ERKENNUNG VON KREBS
UTILISATION DES PROPRIÉTÉS MÉCANIQUES DES CELLULES DANS LES MÉTHODES DE DÉTECTION DU CANCER

(30) Priority: 11.10.2019 US 201962913812 P
(43) Date of publication of application: 17.08.2022
(73) Proprietor: YISSUM RESEARCH DEVELOPMENT COMPANY OF THE HEBREW UNIVERSITY OF JERUSALEM LTD, 9139002 Jerusalem (IL)
(72) Inventor: BENNY, Ofra, 9371554 Jerusalem (IL); BRILL KARNIELY, Yifat, Mevasseret Zion (IL)
(74) Representative: Fleuchaus & Gallo Partnerschaft mbB
(86) International application number: PCT/IL2020/051089
(87) International publication number: WO 2021/070187

(56) References cited:
- JAIDEEP S. DUDANI ET AL: "Pinched-flow hydrodynamic stretching of single-cells", LAB ON A CHIP, vol. 13, no. 18, 1 January 2013 (2013-01-01), page 3728, XP055273513, ISSN: 1473-0197, DOI: 10.1039/c3lc50649e
- STERN TAL ET AL: "Rigidity of polymer micelles affects interactions with tumor cells", JOURNAL OF CONTROLLED RELEASE, ELSEVIER, AMSTERDAM, NL, vol. 257, 23 December 2016 (2016-12-23), pages 40-50, XP085092424, ISSN: 0168-3659, DOI: 10.1016/J.JCONREL.2016.12.013
- YIFAT BRILL-KARNIELY ET AL: "Triangular correlation (TrC) between cancer aggressiveness, cell uptake capability, and cell deformability", SCIENCE ADVANCES, vol. 6, no. 3, 15 January 2020 (2020-01-15), pages 1-13, XP055752479, & Yifat Brill-Karniely ET AL: "Supplementary materials for: Triangular correlation (TrC) between cancer aggressiveness, cell uptake capability, and cell deformability", Science Advances, vol. 6, no. 3, 1 January 2020 (2020-01-01) , page eaax2861, XP055752480, DOI: 10.1126/sciadv.aax2861
- Y BRILL-KARNIELY ET AL: "PHYSICAL PROPERTIES OF PARTICLES CAN CONTROL CELL SPECIFICITY", N A N O B I O & M E D 2 0 1 9, 1 November 2019 (2019-11-01), pages 10-10, XP055752180,

## Description

The project leading to this application has received funding from the European Research Council (ERC) under the European Union's Horizon 2020 research and innovation program (grant agreement No 756762).

### TECHNOLOGICAL FIELD

The application generally concerns methods for in vitro detection of cancer cells utilizing a selective particle-based technology.

### BACKGROUND

A growing evidence correlates cancer aggressiveness with the mechanical deformability of tumor cells. Various processes of cancer development require mechanical adjustment of tumor cells to physical environments, such as growth of high pressure tumors, detachment of metastases forming cells from the tumor, their efficient motion and passage through narrow confinements, and also their establishment in a different location. During the epithelial-mesenchymal transition (EMT), epithelial cells lose their cell polarity and cell-cell adhesion and gain migratory and invasive properties to acquire a mesenchymal stem cell phenotype. The generation of force transmitted to the cell from the extracellular matrix (ECM) is associated with signal transduction and can control the cancerous level of cells.

To date, mechanical measurements of cells are mostly done using single cell methods and, in many cases, require microscopy-based image analysis. Common examples include Atomic Force Microscopy (AFM), microplate stretcher, micropipette aspiration, magnetic twisting cytometry and microscopy image analysis. While such measurements are elegant and precise, their main disadvantage is their low throughput, in most cases reaching a range of 100 tested cells. For clinical applications it is critically important to develop robust tests of cell deformability due to the large heterogeneity among cancer cells.

In addition to the known linkage between cell mechanics and cancer potential, in normal cells, it is known that cell deformability is related to the capacity of cells to uptake particles, mainly in engulfment-mediated internalization including phagocytosis and endocytosis.

US Patent No. 9,267,937 concerns a method for screening particles in vivo, for deciding how to design an optimal drug delivery system, by administering the library of particles to an animal, where the particles can migrate to a targeted tissue or cells and recovering first particles that have migrated to the targeted tissue or cells. By a preferred embodiment the particles are also biologically targeted to the cancer cells.

Dudani et al., LAB ON A CHIP (2013), vol. 13, no. 18, page 3728 discloses a mode of hydrodynamic stretching where inertially-focused cells are squeezed in flow by perpendicular high-speed pinch flows that are extracted from the single inputted cell suspension. The pinched-flow stretching method reveals expected differences in cell deformability in two model systems. Furthermore, hydraulic circuit design is used to tune stretching forces and carry out multiple stretching modes (pinched-flow and extensional) in the same microfluidic channel with a single fluid input.

Tal et al., JOURNAL OF CONTROLLED RELEASE (2016), vol. 257, pages 40 - 50 discloses measurements of differences in rigidity of SPM compared with Wet Polymer Micelles (WPM). It was also examined whether the semi-solid form of hydrated SPMs has an effect on the interaction with tumor cells both in mono-layer systems and in multi-layer clusters of cells as spheroids. For that detailed characterization of SPM compared to WPM was performed, including examinations of particle size, stability, drug release kinetics and cell transcytosis, in melanoma A-375 cells. Cell uptake measurements were done using fluorescent signal analysis, FACS and microscopy imaging, showing enhanced abilities of SPMs to penetrate cells and tissues.

### GENERAL DESCRIPTION

Malignancy potential is correlated with mechanical deformability of cancer cells. Ideally, mechanical properties of cells of an individual patient should be included in cancer diagnostics and in precision therapies. However, clinical applications are limited by current knowledge and by lack of reliable and robust cell mechanical tests. In a comprehensive study, the inventors of the technology disclosed herein have found a *Triangular Correlation (TrC)* between cell deformability, phagocytic-like capacity and cancer aggressiveness. A significant application of the *TrC* is that phagocytic measurements can be a surrogate marker for detecting malignancy of cancer cells based on mechanical properties.

The inventors have found that uptake of inert sub-micron and micro-beads, that *do not* have preferential affinity to cancer cells, was massively higher in cancer cells compared with normal originated cells. Moreover, cells with a higher malignancy potential had greater uptake capacity of such beads. Importantly, in a reciprocal approach, the inventors have sorted either human bladder cancer cells or melanoma cells into sub-populations, solely based on their phagocytic capacity. The more phagocytic sub-populations showed elevated phenotypes of cancer aggressiveness *ex vivo* and *in vivo.* The uptake potential was found to be an imprinted feature preserved genetically and enriched over the sorting cycles. A gene expression profile revealed differences in gene sets associated with regulation of cell-cell and extracellular matrix adhesions and epithelial-to-mesenchymal transition. In all cases, enhanced phagocytic and aggressiveness phenotypes were correlated with greater cell deformability. A computational model supported the notion that the uptake capacity can be a marker for malignancy mediated by cell mechanics. This multidisciplinary approach provides the proof of concept that phagocytic measurements can be applied for cancer diagnostics and precision medicine.

Thus, the present invention is based on the finding of a clear link between cancer aggressiveness (generally any cancer), cell uptake capability and cell deformability. Uptake measurements were performed in high throughput methods: Fluorescence-activated cell sorter (FACS) as well as microplate reader spectrometry followed by detailed microscopy to assess particle localization. Uptake capacity of cancer cells was dramatically higher in skin cancer cells as compared to non-cancerous skin cells as evident by a higher percentage of cells featuring uptake and/or a higher number of particles up taken by each cell.

Additional experiments with eight different cancer cell lines demonstrated a non-monotonic dependence on particle size, which can be explained by physical considerations. By comparing cells with varying malignancies originating from primary (prostate) or metastatic sites, the inventors demonstrated that particle uptake was tightly correlated with cell invasiveness *ex vivo,* and also with elevated cell deformability measured by atomic force microscopy (AFM). To determine whether heterogeneity in particle uptake has biological consequences, using FACS sorting, "phagocytic" and "non-phagocytic" sub-populations of cells ("positive" and "negative", respectively) were generated from the same human cell origin: either melanoma or bladder carcinoma cells. Surprisingly, "phagocytic" phenotype was enhanced over generations, suggesting involvement of genetic effectors. Moreover, "positive" cells were found to be more malignant and invasive than the negative ones in *ex vivo* and *in vivo* experiments.

Significant differences were found in the rate and size of tumor growth between the phagocytic and non-phagocytic cells, associated with elevated histological markers of tumor aggressiveness, and genetic upregulation in transcription of EMT and cytoskeleton reorganization genes. AFM experiments showed that the phagocytic cells were more deformable compared to the non-phagocytic cells. Further mechanistic insights were provided by a physical model using the specific cell parameters measured here, and which quantitatively predicted the uptake patterns found in the experiments.

The *TrC* established here leads to the development of a simple diagnostic scheme using uptake measurements as an additional parameter for grading cancer. To date, cancer diagnostics has not taken into consideration mechanical properties of cells as evident by phagocytosis activity, even though they have been proven to be biomarkers of malignant potential. Cell uptake assessments can provide a simple, robust and accurate diagnostic method and add mechanical knowledge to the existing clinical tools. Moreover, finding specific cell uptake selectivity may provide a mean to rationally design individual drug carriers with higher specificity.

The present invention is defined in the appended claims.

Thus, in a first of its aspects, the invention provides a method for determining at least one cancer-related parameter in a tissue/cells sample from a subject, the method comprising determining engulfment/uptake of particles by cells in said sample having been treated or contacted with a particle population comprising at least two different particle sub-populations, each of said at least two particle sub-population being distinguishable from each other in particles of a different physical parameter and a different identifiable barcode, wherein engulfment/uptake by the cells of at least a portion of said particle population, or a preferential engulfment/uptake of at least a portion of a sub-population, or a preferential engulfment/uptake of a combination of sub-populations determines the at least one cancer-related parameter.

Metastatic cells are of extremely low stress state and adopt an unspread, 3D morphology, resulting in several-fold higher uptake than nonmetastatic counterparts. It is known that cell deformability is related to the capacity of cells to uptake particles, mainly in engulfment-mediated internalization including phagocytosis and endocytosis. Given that cancer potential is correlated with cell deformability, and that cell deformability can control uptake capacity, the ability of cells to engulf particles due to mechanical properties of the cells can provide an indication of cancer aggressiveness. Thus, methods of the invention attempt to tie together the mechanical characteristics of cancer cells and their ability to engulf cells for the detection of cancer-related parameters. As such, within the context of the present invention, the term *"**cellular uptake**"* or any lingual variation thereof refers to the ability of certain cells to engulf or uptake particles into the interior of the cells by a mechanism that involves mechanical targeting. Uptake by pathways characteristic of macrophages, astrocytes, dendritic cells or osteoclasts are excluded from the uptake mechanisms relied upon in context of the present invention.

The term *"**uptake**"* should therefore be taken to encompass engulfment, enveloping, internalization, or any other terminology that signifies mechanical based uptake, as known in the art. As used herein, the term is interchangeable with any of engulfment, enveloping and internalization.

In some embodiments, or independently of the aforementioned method of the invention, the invention further provides a method for determining presence of a cancer cell in a tissue or cell sample, the method comprising determining uptake or engulfment of particles by cells in said tissue or cell sample having been treated or contacted with a particle population comprising at least two different particle sub-populations, each of said at least two particle sub-population comprises or consists particles distinguishable or characterized by a different physical parameter and a different identifiable barcode, wherein cells engulfing at least a portion of said particle population or cells demonstrating preferential uptake of at least a portion of a sub-population are cancer cells, provided that the cells are not macrophages, astrocytes, dendritic cells or osteoclasts.

In some embodiments or independently of any of the aforementioned methods, the invention further provides a method for determining presence of a cancer cell in a tissue or cell sample, the method comprising
- providing a tissue sample or a cell sample having been contacted with a particle population under conditions enabling particles uptake by cells in said sample, the particle population comprising at least two different particle sub-populations, each of said at least two particle sub-population being distinguishable (or comprises or consists particles of a different) from each other in a physical parameter and a different identifiable barcode; and
- determining uptake of the particles by the cells, wherein cells engulfing at least a portion of said particle population, or cells demonstrating preferential uptake of at least a portion of a sub-population, or cells demonstrating a preferential engulfment/uptake of a combination of sub-populations are cancer cells, provided that the cells are not macrophages, astrocytes, dendritic cells or osteoclasts.

Also provided is a method for determining presence of a cancer cell in a tissue or cell sample, the method comprising
- treating or contacting the tissue or cells sample *ex vivo* or *in vitro* with a particle population under conditions enabling particles uptake by cells in said sample, the particle population comprising at least two different particle sub-populations, each of said at least two particle sub-population distinguishable (or comprises or consists particles of a different) from each other in a physical parameter and a different identifiable barcode; and
- determining uptake of the particles by the cells, wherein cells engulfing at least a portion of said particle population, or cells demonstrating preferential uptake of at least a portion of a sub-population, or cells demonstrating a preferential engulfment/uptake of a combination of sub-populations are cancer cells, provided that the cells are not macrophages, astrocytes, dendritic cells or osteoclasts.

As noted herein, methods of the invention aim at determining at least one ***"cancer-related parameter"*** that can assist in a medical determination as to the existence of a cancerous tissue and extent of malignancy. Thus, methods of the invention may alternatively be used or configured or stated for determining presence of a cancer cell or cancerous tissue in a tissue- or cell-containing sample; and further alternatively or additionally determine severity and therapeutic aspects relating thereto, as disclosed herein.

The cancer-related parameter or indicator or marker that is identified by methods of the invention can be used to determine qualitative parameters associated with the existence of a malignancy and further to determine whether a drug agent or a particular therapeutic methodology is or may be effective as an anticancer treatment. The cancer-related parameters may be one or more of presence of cancer cells in a sample obtained from a subject; grade or severity of a cancer in a sample identified as cancerous or in a subject diagnosed with cancer; metastasis or invasiveness potential of a cancer in a sample identified as cancerous or in a subject diagnosed with cancer; whether a particular treatment is effective as an anticancer treatment; whether a potential future anticancer treatment is effective against a particular cancer in a subject; whether a particular mode of delivery of a therapeutic agent for a specific cancer or patient is effective; and others.

The identification of the cancer-related parameter and the following assessment may be for clinical purposes, wherein a tissue sample is examined *ex vivo* or *in vitro,* and the assessment may be used for determining a proper anticancer therapeutic treatment or for improving an existing anticancer therapeutic treatment; or for research purposes, wherein a tissue sample or cells may be from a human or animal source, an experimental animal, or from a cancer cell-line.

When provided with such an indication, clinical assessment should become simpler and provide clinically or experimentally relevant input. In some cases, methods of the invention may assist in providing a qualitative YES/NO answer, for instance to whether a sample such as a biopsied tissue is cancerous or healthy (namely if the sample contains a cell or a plurality of cells that are cancer cells), whether the number of cancer cells is decreasing as a result of treatment, whether a candidate drug would be effective to eradicate a specific cancer, and other qualitative answers or may be helpful in providing a quantitative response, for instance as to the grade of the cancer, the aggressiveness of the particular cancer and so forth.

Described herein is a method for determining cancer in a subject, the method comprising any of the methods disclosed herein. Methods for determining cancer may be any one or more selected from (a) physical examination, to identify lumps or other abnormalities that may indicate a tumor; (b) laboratory tests, involving urine and blood tests to identify abnormalities that can be caused by cancer; (c) imaging tests, to examine bones and internal organs in a noninvasive way, utilizing such methods as computerized tomography (CT), bone scan, magnetic resonance imaging (MRI), positron emission tomography (PET) scan, ultrasound and X-ray, and others; and/or (d) biopsy, wherein tissues or cells samples are removed for testing.

Thus, in such a method, a traditional diagnostic means for determining presence or severity of cancer mentioned above, the method may comprise any of the steps of the invention. For example, in a method for determining presence or severity of cancer, the method involving any one or more of (a) physical examination; (b) laboratory tests; (c) imaging tests; and/or (d) biopsy, the method comprises
(I) -determining engulfment/uptake of particles by cells in a sample having been treated or contacted with a particle population comprising at least two different particle sub-populations, each of said at least two particle sub-population being distinguishable from each other in particles of a different physical parameter and a different identifiable barcode, wherein a engulfment/uptake or at least a portion of the particle population, or a preferential engulfment/uptake of at least a portion of a sub-population, or a preferential engulfment/uptake of a combination of sub-populations determines the at least one cancer-related parameter; or
(II) -determining uptake or engulfment of particles by cells in a tissue or cell sample having been treated or contacted with a particle population comprising at least two different particle sub-populations, each of said at least two particle sub-population comprises or consists particles distinguishable or characterized by a different physical parameter and a different identifiable barcode, wherein cells engulfing at least a portion of said particle population, or cells demonstrating a preferential uptake of at least a portion of a sub-population, or cells demonstrating a preferential engulfment/uptake of a combination of sub-populations are cancer cells, provided that the cells are not macrophages, astrocytes, dendritic cells or osteoclasts; or
(III) -providing a tissue sample or a cell sample having been contacted with a particle population under conditions enabling particles uptake by cells in a sample, the particle population comprising at least two different particle sub-populations, each of said at least two particle sub-population being distinguishable (or comprises or consists particles of a different) from each other in a physical parameter and a different identifiable barcode; and
   - determining uptake of the particles by the cells, wherein cells engulfing at least a portion of said particle population, or cells demonstrating a preferential uptake of at least a portion of a sub-population, or cells demonstrating a preferential engulfment/uptake of a combination of sub-populations are cancer cells, provided that the cells are not macrophages, astrocytes, dendritic cells or osteoclasts; or
(IV) -treating or contacting the tissue or cells sample *ex vivo* or *in vitro* with a particle population under conditions enabling particles uptake by cells in a sample, the particle population comprising at least two different particle sub-populations, each of said at least two particle sub-population distinguishable (or comprises or consists particles of a different) from each other in a physical parameter and a different identifiable barcode; and
   - determining uptake of the particles by the cells, wherein cells engulfing at least a portion of said particle population, or cells demonstrating a preferential uptake of at least a portion of a sub-population, or cells demonstrating a preferential engulfment/uptake of a combination of sub-populations are cancer cells, provided that the cells are not macrophages, astrocytes, dendritic cells or osteoclasts.

In any method of the invention, determination of the at least one cancer-related parameter involves treating or contacting a tissue sample or a cell sample, that may be a cancerous tissue, or a tissue suspected of being cancerous, with a population of particles and determining uptake of the particles by the cells or cells in the tissue. The sample may be one obtained from any organ or tissue of a subject. This includes the skin, any internal tissue or organ, or any tissue which may be cancerous, as known to a person of skill in the art. Uptake by the cells, the rate of uptake, and/or the preferential uptake of a specific group of particles provides an indication as to the presence of cancer cells, the severity or aggressiveness of the cancer, metastatic potential, resistance to drugs and low levels of oxygen; or the absence of a malignancy. For achieving both qualitative and quantitative assessments, the cells or tissue are contacted with a population of particles that comprises at least two different sub-populations of particles. Each sub-population differs from another in (a) at least one physical parameter; and (b) in at least one identifiable tag or barcode feature that can be used to distinguish one sub-population over another (after particles have been taken up by the cells). The tendency of a particular healthy tissue or cell (a normal cell) to uptake a specific population of particles is determined ahead of time and can be used as a ***"fingerprint"*** or a control to determine whether a sample containing the same tissue or cells is indeed healthy (namely not containing a cancer cell), or cancerous (namely containing a cancer cell). For example, a fingerprint defining an uptake by healthy cells of a particle population comprising three different particle sub-populations such that only (or a preferentially) particles of the smaller size rather than of medium or larger sizes are engulfed by the healthy cells; or spherical particles rather than rod-shaped particles are engulfed by the healthy cell, may be used to distinguish from a sample containing cells which uptake of the same particle population would yield an uptake profile that is different, i.e., uptake of particles of larger sizes or non-spherical particles.

Since uptake of particles was observed to be massively higher in cancer cells as compared with normal cells, and as cells with a higher malignancy potential had greater uptake capacity of such particles, cells "... ***engulfing at least a portion of said particle population or cells demonstrating a preferential uptake of at least a portion of a sub-population...",*** as used herein, are regarded cancer cells that can be differentiated from healthy cells. As some classes of healthy cells are known to have internalization or engulfing capabilities, uptake of particles by cells such as macrophages, astrocytes, dendritic cells or osteoclasts is not considered in determining an end result. The engulfment/uptake of particles by the cells need not result in the internalization of the complete population or sub-population of particles. For purposes herein, it is sufficient to have only a 'portion' of said population engulfed or taken up by the cells. Thus, the expression "***at least a portion***" in reference to said particle population or to a sub-population refers to engulfment or uptake of any number of particles. Any number of engulfed or taken up particles is sufficient for the determination of a cancer-related parameter or for the purpose of determining presence or absence of a cancer cell in a sample. In some embodiments, the number of parties may range from a few to very many, or from 1 particle per cell to 0.1, 0.2, 0.3, 0.4, 0.5, 1, 1.5, 2 % or more of the cells. The size of the population portion that is engulfed depends, *inter alia,* on the type of cell, on whether it is a heathy cell or a cancer cell, on the physical attributes of the particles and others.

The term "***preferential uptake***" generally refers to a tendency of cells to uptake one sub-population over the other due to a physical parameter that distinguishes the sub-population from other sub-populations in the sample. The preferential uptake may be determined qualitatively by identifying the particles that have been taken up by the cells, or quantitatively by determining the percentage of cells that have taken up particles, or the number of particles that were taken up by each cell or a combination of the above, number of engulfed particles from one sub-population as compared to another sub-population. In some embodiments, cells may be regarded cancerous if their uptake, as compared to the same type of healthy cells, is statistically significant. In other embodiments, a cell may be regarded cancerous if the number of particles engulfed by the cell is 2, 3, 4, 5, 6, 7, 8, 9, 10, 20, 30, 40, 50, 60, 70, 80, 90, 100... 10,000 times greater as compared to the number of cells engulfed by a healthy cell.

Due to the preferential uptake of cancer cells as compared to healthy cells (preferably of the same type) over the period during which the cells are contacted with the particles, e.g., being between 4 and 24 hours, uptake of particles by healthy cells that are different from macrophages, astrocytes, dendritic cells and osteoclasts is negligible or nonexistent.

The "***at least one physical parameter***" that distinguishes one sub-population over another is at least one parameter relating to the shape and/or size and/or deformability capacity of the particles. The at least physical parameter does not therefore relate to the material composition of the particles or to any parameter that defines the particles chemical or biological affinity to a particular tissue or cell type. In other words, the particles are selected to be of different shapes and/or sizes and/or deformability (elasticity), but generally of the same chemical or biological affinity to healthy and cancer cells.

The at least one physical parameter may be one or more of particles size, particle shape and particle stiffness (or elastic deformability).

Thus, a population of particles used according to the invention comprises at least two, or two or more, or a plurality of particles sub-populations, wherein each of the sub-populations comprises particles of the same physical parameter. Namely, particles within each of the sub-populations will be identical, i.e., have the same size and shape. Each sub-population, however, differs from another in at least one physical parameter, e.g., size, shape or stiffness/flexibility/deformability, such that the population of particles will comprise two or more different types of particles. In cases where one sub-population shares particle size with another sub-population, the two sub-populations will differ in particles shape. Similarly, in cases where one sub-population shares particle shape with another sub-population, the two sub-populations will differ in particle size.

Each particle population used in accordance with the invention is thus a mixed particles population that comprises two or more different types of particles, as explained herein. The number of sub-populations may be tailored based on the particular cancer-related parameter which determination is desired, based on any parameter relating to the tissue or cells to be assessed, or based on the particular cancer which detection or assessment is desired. In some embodiments, the particle population comprises between 2 and 10 sub-populations, or between 2 and 9, 2 and 8, 2 and 7, 2 and 6, 2 and 5, 2 and 4, or 2 or 3 sub-populations. In some embodiments, the number of sub-populations is 2, 3, 4, 5, 6, 7, 8, 9 or 10. In some embodiments, the number of sub-populations is 2, 3, 4, 5 or 6.

Each sub-population differs from another in the population of particles in size. The size of the particles may be measured or stated as one typically would in the field of material sciences or nanotechnology or in any other relevant field. For elongated particles, the size may be of the particle's longest axis, e.g., length. For spherical particles, the size may be the diameter of the particle. For circular particles, the size may be the diameter of the particle or its thickness. When reference is made to particles of nanometric or micrometric dimensions or sizes, each of the particles' dimensions is nanometric or micrometric, respectively, or at least the longest axis of the particles is in the indicated dimension.

One or more of the sub-populations may comprise particles having a size or a diameter in the nanometric range and one or more sub-populations may comprise particles having a size or a diameter in the micrometric range.

All particles in a particle population may be nanometric in sizeor micrometric in size.

A population of particles may thus comprise a sub-population having a particle size (length or diameter) between 300 nm and 800 nm, another sub-population comprises particles having a size between 800 nm and 1.5 micron and a further sub-population comprises particles having a size between 1.5 micron and 4 microns.

The particle population may similarly comprise a sub-population having a particle size (length or diameter) between 300 nm and 500 nm, another sub-population comprises particles having a size between 500 nm and 1.5 micron and a further sub-population comprises particles having a size between 1.5 micron and 4 microns.

Alternatively, the population may comprise a sub-population having a particle size (length or diameter) below 500 nm, another sub-population comprises particles having a size between 500 nm and 1 micron, and a further sub-population comprises particles having a size greater than 1 micron.

In order for the sub-populations to be sufficiently distinct where size is the physical property distinguishing between the various sub-populations, the size distribution of particles in each sub-population should be narrow. As used herein, "size distribution" refers to a single value that is representative of the distribution, e.g., a central tendency of particle size (length or diameter across the distribution), such as an arithmetic mean, weighted mean, midrange, midhinge, trimean, Winsorized mean, median, or mode of the distribution is representative of the sub-population. The size distribution is said to be "narrow " to reflect particles within a sub-population that is of a size distribution which is substantially monodisperse. The coefficient of variation (percent CV) of particles in each sub-population is at most 5%, or at most 4%, 3%, 2%, 1%, 0.5% or lower than 0.5% (values being inclusive).
the sub-populations may differ from one another in particle shape. Any particle shape may be useful in accordance with the invention. In some embodiments, the particles may be symmetric or asymmetric and may be further selected amongst circular or spherical shaped particles, disc shaped, round particles, elongated particles and polygons of various shapes. In some embodiments, the particles are in a shape of spheres, rods, stars, cubes, polygons and others.

The sub-populations may differ from one another in particle deformability, namely in the particles ability to undergo elastic deformation in response to an external stress or force that is imposed, for example, by a cell membrane. In some embodiments, the population of particles comprises particles having a Young's modulus ranging between 1kPa and several thousand kPa. The deformability may be characterized by a Young modulus of between 1 and 10,000kPa, or between 1 and 1,000, 1 and 900, 1 and 850, 1 and 800, 1 and 750, 1 and 700, 1 and 650, 1 and 600, 1 and 550, 1 and 500, 1 and 450, 1 and 400, 1 and 350, 1 and 300, 1 and 250, 1 and 200, 1 and 150, 1 and 100, 1 and 90, 1 and 85, 1 and 80, 1 and 75, 1 and 70, 1 and 65, 1 and 60, 1 and 55, 1 and 50, 1 and 45, 1 and 40, 1 and 35, 1 and 30, 1 and 25, 1 and 20, 1and 15, or between 1 and 10 kPa.

The particles are selected to having a Young's modulus ranging between 0.1 and 10,000kPa, or between 0.1 and 1,000, 0.1 and 900, 0.1 and 850, 0.1 and 800, 0.1 and 750, 0.1 and 700, 0.1 and 650, 0.1 and 600, 0.1 and 550, 0.1 and 500, 0.1 and 450, 0.1 and 400, 0.1 and 350, 0.1 and 300, 0.1 and 250, 0.1 and 200, 10. and 150, 0.1 and 100, 0.1 and 90, 0.1 and 85, 0.1 and 80, 0.1 and 75, 0.1 and 70, 0.1 and 65, 0.1 and 60, 0.1 and 55, 0.1 and 50, 0.1 and 45, 0.1 and 40, 0.1 and 35, 0.1 and 30, 0.1 and 25, 0.1 and 20, 0.1and 15, or between 0.1 and 10 kPa.

As used herein, the terms deformability, elasticity, stiffness and flexibility are interchangeable.

Typically, particles used in accordance with the invention are neutral in charge. However, for some applications, and depending, *inter alia,* on the particle composition and coating, and also on the tissue to be assayed, the particles may be provided positively charged, thus exhibiting a higher affinity to most cells, or negatively charged, thus exhibiting a lower affinity to most cells. In some embodiments, the particles in all sub-populations are neutral. In some other embodiments, all particles in a particle population have the same charge (positive or negative) but differ based on the sub-populations (in size, shape or deformability).

In some embodiments, the sub-populations differ from each other in particle size or particle shape. In some embodiments, the sub-populations differ from each other in particle size and particle shape. In some embodiments, the sub-populations differ from each other in particle size or particle deformability. In some embodiments, the sub-populations differ from each other in particle size and particle deformability. In some embodiments, the sub-populations differ from each other in particle size, particle shape and particle deformability.

Irrespective of any of the aforementioned physical parameters that define the particles, the particles are selected to be biocompatible to contacting with cells or tissues, for both *in vitro* or *ex vivo* applications.

The particles should preferably be inert and non-toxic to the cells, tissue or the subject. The particles should also be of a material that is not recognize by, or that does not bind to cells such as receptors, pumps, transporters, channels and others, so that their uptake is passive and is affected only by their physical properties, e.g., size, shape, stiffness and not by any material property or functionality that is present or is part of the particle composition.

Particles used may be polymeric particles. The polymeric particles may be composed of a material selected from polyesters, polyamides, polycarbonates, polycarbamates, polyacrylates, polystyrene, polyureas, polyethers, polyamines, polyanhydrides, poly(hydroxyacids), poly(lactic acid), poly(glycolic acid), poly(orthoesters), polyphosphazene, ethylene-vinyl acetate copolymer, polyurethanes, polyacrylates, polymethacrylates, polyacrylonitriles, poly(amidoamine) dendrimers, poly(L-lactide-co-L-lysine), poly(serine ester), poly(4-hydroxy-L-proline ester), maleimide-poly(ethyleneglycol)-block-poly(D,L-lactic acid), COOH-poly(ethyleneglycol)-block-poly(D,L-lactic acid), methoxypoly(ethyleneglycol)-block-poly(D,L-lactic acid), proteins, polysaccharides, PEGylated poly(hydroxy acids), PEGylated poly(orthoesters), poly(caprolactone), PEGylated poly(caprolactone), polylysine, PEGylated polylysine, poly(ethylene imine), PEGylated poly(ethylene imine), and combinations thereof.

The particles may be selected from polystyrene, PLGA and PEG based.

The particles may be polystyrene particles.

The particles may be PLGA particles.

The particles may be PEGylated particles. Such may be selected form PEGylated poly(hydroxy acids), PEGylated poly(orthoesters), poly(caprolactone), PEGylated poly(caprolactone), polylysine, PEGylated polylysine, poly(ethylene imine), PEGylated poly(ethylene imine) and others.

Particles used in accordance with methods and products of the invention are solid particles constructed of a material as defined and selected herein. As particles are not intended for delivering an agent into a cell, but rather as a tool for defining mechanical properties of a cell and a respective cancer-related parameter, the particles are not in a form of a liposome, vesicles of any type, lysosomes, micelles or reverse micelles. Particles that can decompose or erupt upon engulfment by the cells are also excluded in some specific embodiments of the invention.

Sub-populations making up particle populations used in accordance with the invention are also distinguishable by at least one identifiable tag, label, tracer or a barcode feature that is appended to particles of each sub-population. This "***barcode feature***" is a tag, a label or a tracer moiety that enables selective tracing and identification of the various sub-populations. In other words, the barcode feature enables tracing of (finding position or location) and distinguishing between one sub-population over another, and thus enables determination, for example, of which particle sub-population has been taken up by the cells or tissues and which have not. The barcode feature is provided in a form of a chemical moiety that is present on a surface region of the particles, or in a form of a material that is comprised within the particles (a particle composition) and which detection by physical or chemical means is possible. The chemical moiety provides a ***distinguishable signal*** that distinguishes the particle sub-population from others, which enables positioning of the particle out of or within a cell, and which enables independent quantification. In some embodiments, the chemical moiety can contain an element or a functionality that provides the distinguishable signal in response to at least one detection means. The signal may be detected by any means known in the art, which may include spectroscopic means, chemical means, biological means or any other qualitative or quantitative means as known in the art. Exemplary tagging moieties and methods for their association with, e.g., particles of various compositions and shapes are known in the art. See for example Chem. Soc. Rev., 2015, 44, 4743-4768 "An overview of nanoparticles commonly used in fluorescent bioimaging".

For example, a chemical moiety may be in a form of a luminescent tag, a photoluminescent tag, a chemiluminescent tag, a fluorescent tag (such as Cy5 and Cy3), a magnetic tag, an MRI tag, a colorimetric tag, a chemical tag having reactivity towards certain agents (or when contacted with some agents), a hybridization tag, a polynucleotide tag, a peptide tag, a semiconductor tag (such as a particle), XRF tag, an X-ray tag, quantum dots and others. Each of these tags may be detected by suitable technologies known in the art, including for example, FACS, spectrometers, fluorimeters, microplate readers, microscopies, amplification or hybridizations on a chip, MRI, XRF readers, X-ray readers and others.

In some embodiments of the invention, the particles in accordance with the invention are polyester particles conjugates or associated with at least one fluorescent tag. The fluorescent tag may be any material that absorbs light energy of a specific wavelength and re-emits light at a longer wavelength. The fluorescent tag may be selected from any such material, exemplified by the chemical families xanthenes, cyanines, squaraines, naphthalenes, coumarins, oxadiazoles, anthracenes, pyrenes, oxazines, acridinea, Arylmethines, tetrapyrroles and dipyrromethenes.

Examples of fluorescent tags include hydroxycoumarin, aminocoumarin, methoxycoumarin, cascade blue, pacific blue, pacific orange, lucifer yellow, NBD, R-Phycoerythrin, fluorescein, G-Dye100, G-Dye200, G-Dye300, G-Dye400, Cy2, Cy3, Cy3B, Cy3.5, Cy5, Cy5.5, Cy7, Rhodamine, Lissamine Rhodamine, Texas Red, allophycocyanin and others.

In some embodiments, the fluorescent tag is Cy5 or Cy3.

In accordance with methods of the invention, a tissue or a cells sample is contacted or treated with a particle population. In most applications, the tissue or cell sample is contacted with a dispersion of particles in a liquid medium. In some cases, the sample is contacted with the particles by placing the particles or a medium containing same on the tissue or cells. Alternatively, contacting of the sample with the particles may be by flowing the particles over the sample.

In some embodiments of methods of the invention, the methods comprise obtaining a ***sample,*** such as a tissue sample or a cell sample known to be cancerous or suspected of being cancerous or selected for determining the presence or absence of a cancer cell. The tissue or cells may be derived from a healthy subject, a subject diagnosed with having cancer or an experimental sample from an unknown source. The subject may be a human subject or a non-human subject. The sample may be in a form of cells obtained from a living subject, e.g., when provided in a suspension or present on a surface or a substrate, optionally with known fixed characters; in a form of a fluid sample or a solid biopsy obtained from a subject; in a form of an artificially produced aggregate formed from cancer cells (spheroids, organoids, tissue fragments).

The cells may be derived from any tissue or organ of a subject. The cells may be epithelial in region; blood cells; bone marrow cells; cells of the immune system; cells originating from connective tissues such as fat, muscle and bone; cells of the central nervous system, including the brain and spinal cord; cells originating in the lining of body cavities; and others.

In some embodiments, the sample obtained is pre-treated before being contacted with the particle population. In other words, methods of the invention comprise a step of pre-treating a tissue or cell sample or a step of obtaining a treated sample. In a step of pre-treatment, a sample is treated to eliminate cells having natural phagocytic properties. The cells may be macrophages (in cases where the sample is a blood sample or comprises blood), astrocytes (in cases where the sample is a brain tissue or baron cells or one which comprises such), dendritic cells (for example in skin samples) and osteoclasts (from bone samples). In case a method of the invention comprises a step of obtaining a treated sample, such a sample would be free of macrophages, astrocytes, dendritic cells and osteoclasts.

Alternatively, a sample containing such cells may be used without further treatment and any signal resulting from presence of such cells may be eliminated by reducing the signal generated from these cells based on a control signal indicative of their presence.

Thus, in some embodiments, a sample assayed according to methods of the invention comprises cells, excluding macrophages, astrocytes, dendritic cells and osteoclasts.

Sample pre-treatment may be achievable by treating the sample with agents that selectively eliminate macrophages, astrocytes, dendritic cells and osteoclasts, as known in the art. For example, phagocytic cells may be removed by particles with bisphosphates.

In some embodiments, methods of the invention comprise obtaining a particle population, as disclosed herein.

In some embodiments, methods of the invention comprise contacting a tissue sample or a cell sample with a particle population.

Irrespective of the means by which the sample is contacted with the particles, the conditions under which the contacting is carried out are sufficient to permit, enable or drive uptake of the particles by the cells. Such conditions may be selected amongst temperature, ionic strength, pH, and reagents. Typically, the cell sample or tissue sample to be assayed is maintained under conditions suitable to maintain the cells viable. These conditions include use of a cell culture, as known in the art, maintained at 37°C for a period between several minutes to 24 hours, in a medium or a buffer compatible for cells. Typically, within the first 4 hours uptake of particles is observed. To eliminate non-specific adherence, following particle uptake, cells are maintained at 4°C to eliminate levels of adhesion on surface versus cell uptake.

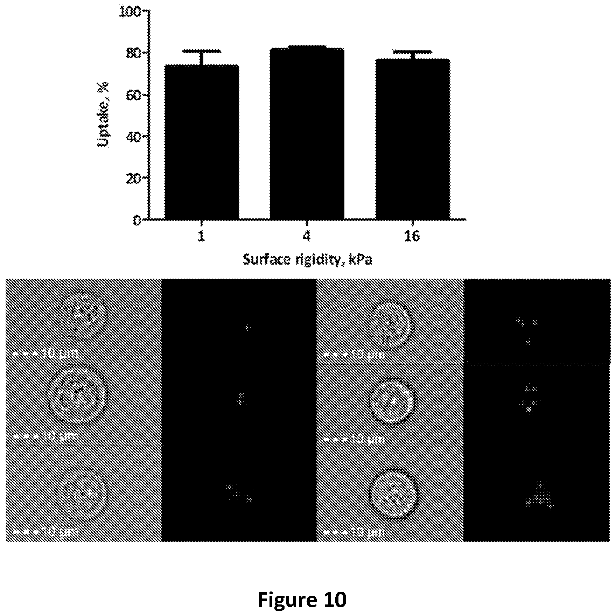

Once particles have been engulfed or taken up by the cells, cell sub-populations may be distinguishable by detecting a distinguishable signal indicative of a certain particle sub-population. Thus, following uptake, the sample is read by any of the means mentioned herein to determine uptake by the cells, determine signal intensity indicative of degree or extent of uptake (how many particles have been taken up by single cell) and identify an ***uptake profile*** that can be compared to a fingerprint obtained for a healthy tissue or control metastatic samples using the same particle population. The comparison between the uptake profile of the tested sample and the fingerprint profile obtained for a control (healthy or cancerous cells) can be used to determine presence or absence of a malignancy, severity thereof and other factors relating to the cancerous cells in particular metastatic potential. The uptake profile may be in a form of a histogram describing particle interaction with cells, quantifying the number of particles that have been taken up by a cell (particles per cell). An example of such a histogram is shown in Fig. 10A.

Thus, methods of the invention directed at identifying at least one cancer-related parameter or at determining presence of a cancer cell in a tissue or cell sample comprise
- determining uptake or engulfment of particles by cells in said tissue or cell sample having been treated or contacted with a particle population comprising at least two different particle sub-populations, each of said at least two particle sub-population comprises or consists of particles having or characterized by a different physical parameter and a different identifiable barcode, as disclosed herein; and
- determining by any one or more of the methods disclosed herein an uptake profile relating to the particle population or plurality of sub-populations and/or the amount or number of particles taken up by the cells;
- comparing said uptake profile to a fingerprint obtained for heathy cells utilizing a particle population identical to the particle population with which the sample was contacted;

wherein a deviation from the fingerprint is indicative of one or more of (i) presence of a cancer cell in the sample, (ii) absence of a cancer cell, and (iii) metastatic potential (or malignancy or aggressiveness of cells), namely the tendency of a primary tumor to form secondary metastatic lesions, as known in the art. The term ***"metastatic potential"*** encompasses further malignancy or aggressiveness potential.

In some embodiments, the method comprises providing a tissue sample or a cell sample having been contacted with a particle population under conditions enabling particles uptake by the cells, the particle population comprising at least two different particle sub-populations, each of said at least two particle sub-population comprises or consists particles of a different physical parameter and a different identifiable barcode.

In some embodiments, the method comprises treating or contacting the tissue or cells sample *ex vivo* or *in vitro* with a particle population under conditions enabling particles uptake by the cells, the particle population comprising at least two different particle sub-populations, each of said at least two particle sub-population comprises or consists particles of a different physical parameter and a different identifiable barcode.

As noted herein, the **"*deviation from the fingerprint*"** that is indicative of one or more of (i) presence of a cancer cell in the sample, (ii) absence of a cancer cell, and (iii) metastatic potential or malignancy or aggressiveness of cells, may be any one of the following:
(a) A change in the particles sub-population that is taken up by the cells (e.g., in case healthy cells have propensity to uptake particles of sub-population A, while the cells in the sample demonstrate a propensity to uptalking particles of sub-population B);
(b) A change in the relative uptake of one sub-population over the other;
(c) A change in the amount/number of particles being taken up by the cells;
(d) A change in the relative amounts of particles of different sub-populations that are taken up by the cells;
(e) A change in the type of cells that uptake one or more of the sub-populations;
(f) A change in the uptake profile by certain types of cells; and/or
(g) A change in the diversity of particles taken up by the cells.

The measured results from the detection of the barcode can provide a qualitative indication as to the presence of a cancerous cell or tissue or a quantitative measure of the level/amount of particles taken up by the cells as a measure of grade or severity or metastatic potential or the cancerous cells or tissue. The quantification of the results may be achieved by comparing the readings to calibration curves developed by assaying healthy cells or cancer cells with known properties (grade, severity, metastatic potential etc.). Analysis can be done using machine learning, as known in the art.

In some cases, cells or tissues which have engulfed particles are separated from cells or tissues which do not exhibit particle uptake. In some embodiments, the tissue or cell sample is washed in order to separate cells which have not engulfed particles from cells or tissues in which particles uptake was evident. In other words, the tissue or cells are washed to separate healthy cells from cancerous cells. The various cells may be distinguishable by the presence of the particles different barcode features.

Methods of the invention may be used alone as standalone cancer assessment procedures or in conjunction with other detection or cancer assessing methods. The cancer which presence and severity are to be assessed using methods of the invention may be any cancer known in the art. The cancer may be selected from blastoma, carcinoma, lymphoma, leukemia, sarcoma, mesothelioma, glioma, germinoma, choriocarcinoma, melanoma, glioblastoma, lymphoid malignancies and any other neoplastic disease or disorder.

The cancer may be squamous cell cancer (e.g. epithelial squamous cell cancer), lung cancer including small-cell lung cancer, non-small cell lung cancer, adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, skin cancer as well as head and neck cancer.

The cancer may be a solid tumor cancer (such as breast cancer, prostate cancer, sarcomas and skin cancer) or a cancer of a hematopoietic line (cancers of blood, bone marrow, lymph and lymphatic system).

Described herein is a tool for carrying out a method according to the invention. The tool may be in a form of a diagnostic device comprising a chamber or a region for holding a tissue/cell sample and means for bringing a dispersion or suspension of particles into contact with the sample.

The tool may be in the form of a microfluidic device or a flow chip. The tool may be fabricated using advanced techniques that include, microfabrication or 3D printing.

Also disclosed herein - but not covered by t he claims - is a kit for use in the detection of cancer, or in carrying out methods of the invention, the kit comprising a composition of at least two particles population, optionally carried in a liquid medium, as defined herein, and instructions of use.

The kit may further comprise standard calibration curves and computer program analysis.

### BRIEF DESCRIPTION OF THE DRAWINGS

In order to better understand the subject matter that is disclosed herein and to exemplify how it may be carried out in practice, embodiments will now be described with reference to the accompanying drawings, in which:

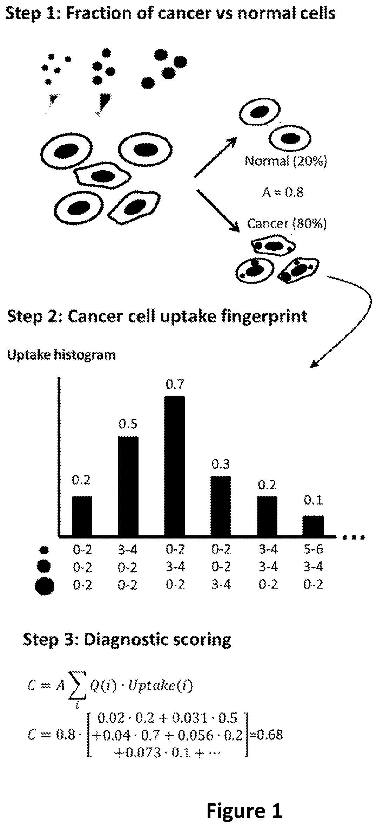

**Fig. 1** depicts a scheme of optional steps in cancer scoring based on cell uptake fingerprint. In the first step there is fractioning between normal and cancer cells. Then in step 2 there is a detailed analysis of the cancer cells fingerprint. This is given here by a multidimensional histogram showing the fraction of cells which internalized a defined number of particles from each type. In step 3 a formula is used to provide the diagnostic score *C,* which captures information on the percent of malignant cells in the population and on the mechanical signatures of the cells, accounting for cross-correlation in the uptake patterns.

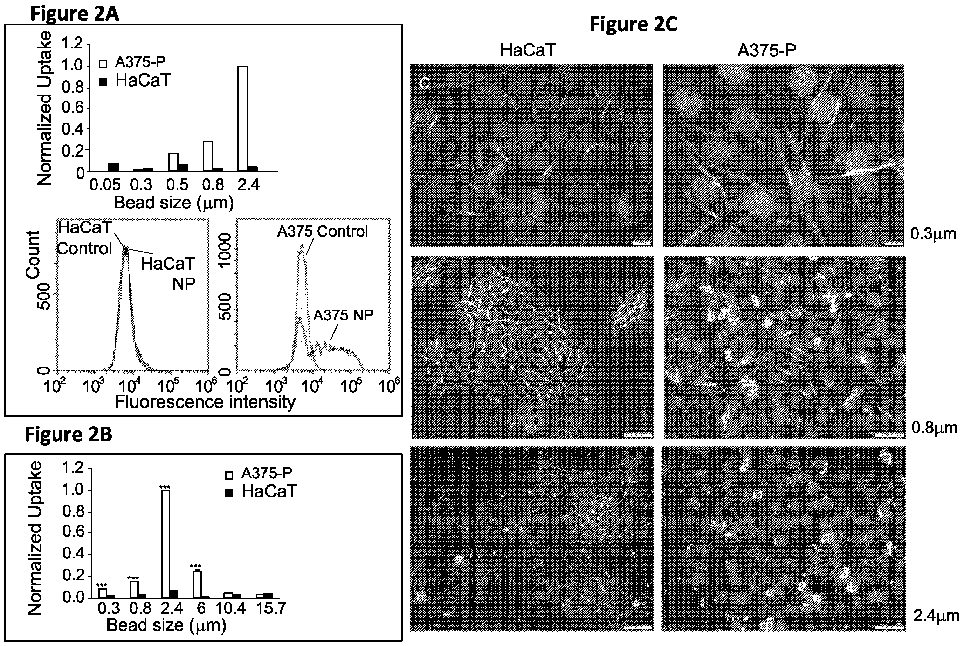

Figs. 2A-C demonstrate that cancer skin cells (A375-P) present an enhanced uptake compared with non-cancer skin cells (HaCat). **(A)** FACS analysis is presented as column plot of normalized average uptake values of 0.05, 0.3, 0.5, 0.8, 2.4 µm fluorescently labeled polystyrene beads. Representative histograms for 2.4 µm beads are shown at the bottom. **(B)** Microplate reader fluorescence analysis. Excitation: 530/25 Emission: 590/20, with 0.3, 0.8, 2.4, 6, 10.4, 15.7 µm beads. The unit-less results in the column plots of panels A and B are normalized by the intensity of particle fluorescence to represent the relative mass of cell internalized particles, and are scaled to a maximal value of 1. **(C)** Fluorescent microscopy images of cells incubated with 0.3, 0.8, 2.4 µm beads. Blue: DAPI, Red: beads. Bar = 10 µm (upper panels, 0.3 µm) or Bar = 50 µm (middle and lower panels, 0.8 and 2.4 µm).

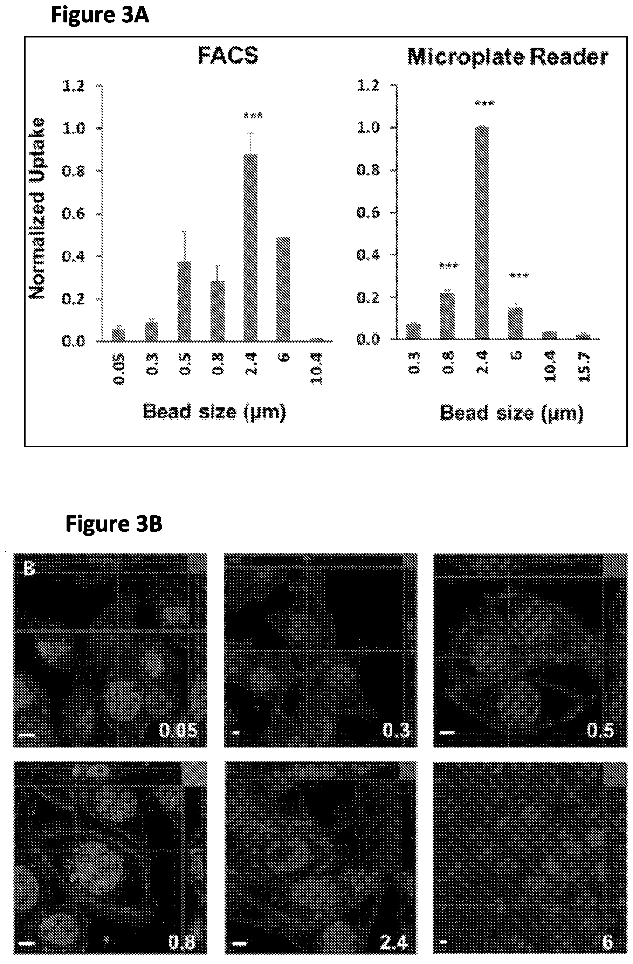

Figs. 3A-F demonstrate non-monotonic dependence of cancer cell uptake on bead size. Cancer cells interaction with inert spherical fluorescent polystyrene beads increases with bead size, reaching a maximum around 2.4 µm bead diameter and then decreases: **(A)** FACS analysis (left) average normalized uptake over 8 cell lines: A375-p, A375-SM, PC3M-P, PC3M, LN4253J, 253J-B5, SN12, SN12-C. Microplate fluorescent analysis Excitation: 530/25 Emission: 590/20 of A375-P, A375-SM, PC3M-P, PC3M-LN4. **(B)** Confocal fluorescent images (Blue - DAPI, Green - FITC-Phalloidin, Red polystyrene fluorescently labeled beads), Bar = 10 µm. **(C)** Physical model predicted the non-monotonic dependence due to the interplay between adhesion and cell deformability. **(D)** The free energy of the wrapping model has a minimum both as a function of the bead radius and the wrapping angle, ϑ (Panel D, bottom), however for small beads of R<Rc there is no stable adhesion (Panel D, top, plots downwards are for R= 0.05, 0.11, 0.14 (Rc, dashed), 0.18, 0.23 µm). (**E**) The stable adhesion angle, *ϑ̋* (rad) is shown vs. Young's modulus and R. (**F**) model geometry. Parameters used in plots of panels D-E are based on AFM calculations in PC3M-LN4 cells.

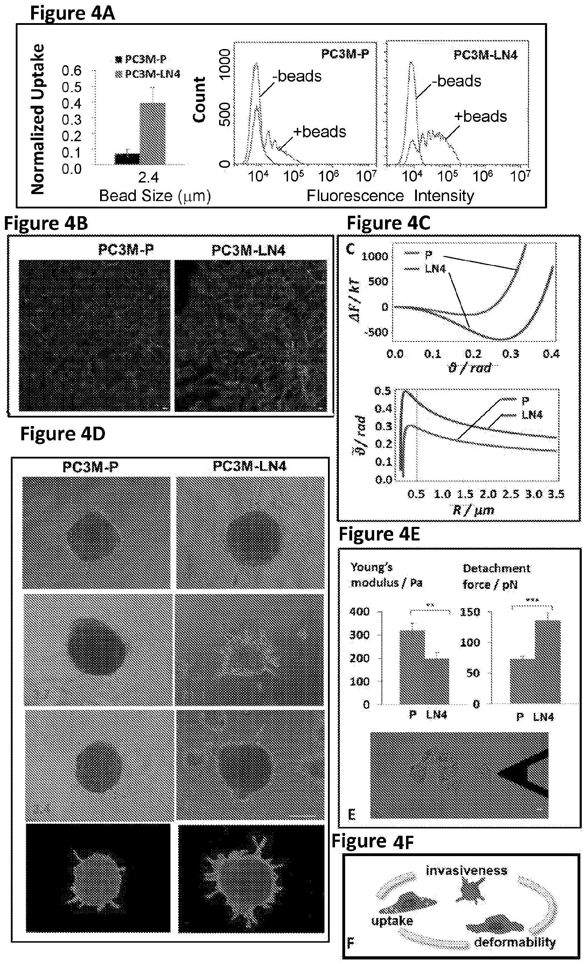

Figs. 4A-F depict TrC in primary vs metastatic cancer cells. **(A-C)** Uptake comparison of 2.4 µm beads in primary vs metastatic cell lines of prostate cancer, PC3M-P and PC3M-LN4, respectively, showing elevated uptake by the metastatic cells: **(A)** FACS analysis, **(B)** confocal images, Bar= 10 µm and **(C)** results of the theoretical model with specific parameters of PC3M-P and PC3M-LN4: Δ*F* (ϑ) curves for R=2.37 µm (top) as well as *ϑ̋*(R) (bottom) demonstrate that the metastatic cells internalize more particles. **(D)** Spheroid invasion test shows enhanced invasiveness of the metastatic compared to the primary cells. Compactness values are shown in red. At the bottom shown are examples of image analysis made for calculating the compactness of the microscope snapshots above them. Bar= 100 µm. **(E)** A comparison of the mechanical properties of the cells: Young's modulus and the maximal adhesion force, using AFM, shows that the metastatic cells are more elastic and more adhesive than the primary cells. A typical snapshot of a PC3M-LN4 cell from the AFM microscope is shown. Bar= 10 µm. The results presented in panels A-E support the TrC as shown in the cartoon **(F).** The PC3M-LN4 cells have higher uptake capacity (Panels A-B), they are more invasive (Panel D) and are more deformable (Panel E) in comparison with the PC3M-P cells, as also supported by the physical model in Panel C.

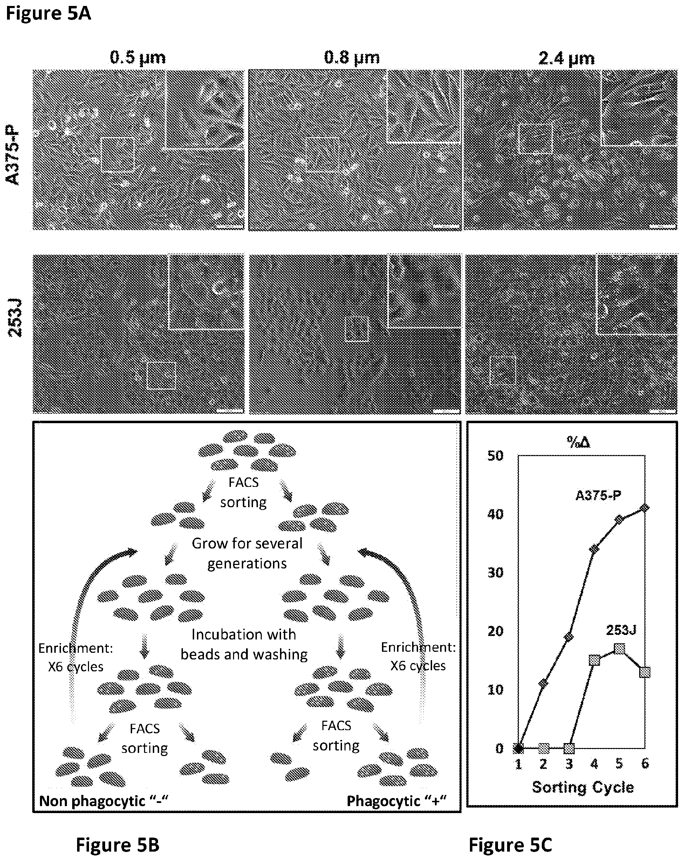

Figs. 5A-C depict Sorting cancer cells based on uptake capacity of 2.4 µm beads. (A) Uptake is heterogenic among a cell population. Fluorescence microscope images of either A375-P or 253J cell types after incubation with beads show that while some cells internalized many particles others did not internalize any. Scale bar of upper images is 50 µm, and of lower ones is 100 µm. **(B)** Scheme of FACS sorting procedure for separating cells based on uptake capacity. Each sorted cycle resulted in two sub-populations of phagocytic ("positive") cells and non-phagocytic ("negative") cells. **(C).** Enrichment uptake graph. Δ is the percent of positive cells generated from a previously positive population, minus the percent of positive cells generated from a previously negative population.

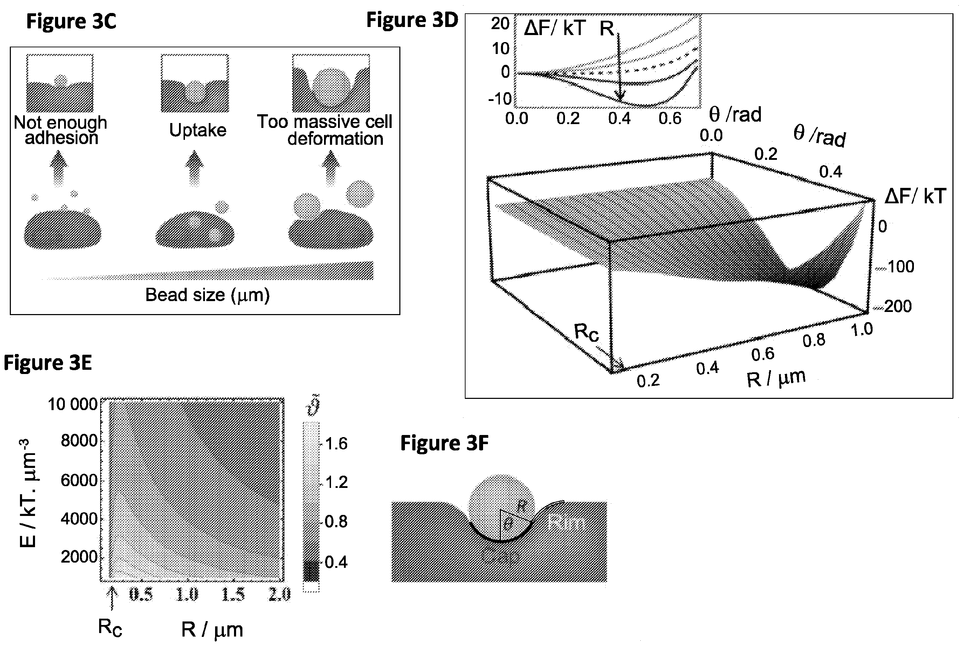

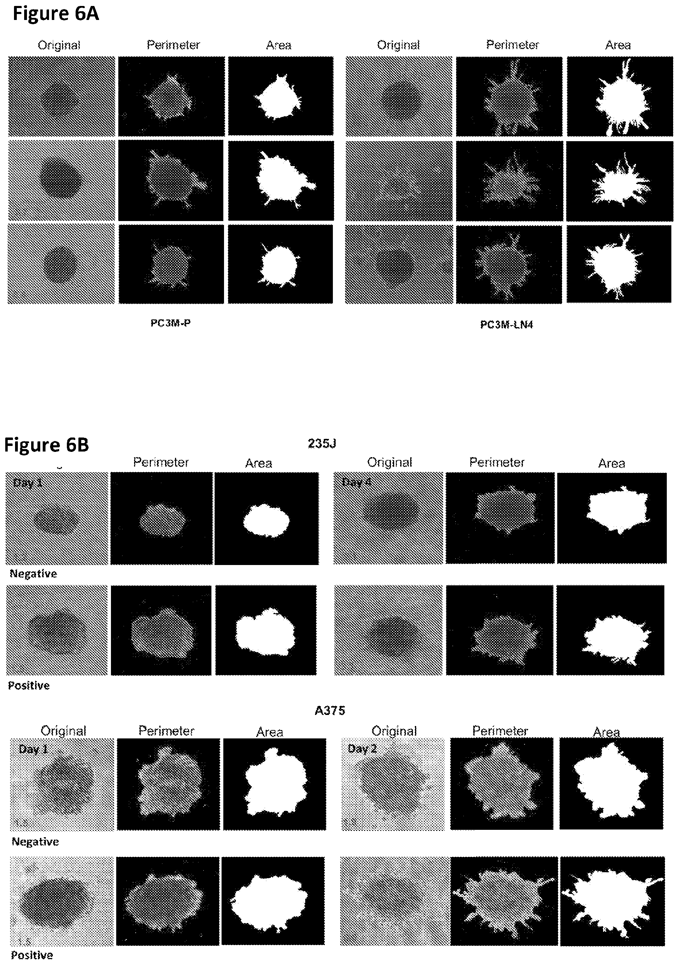

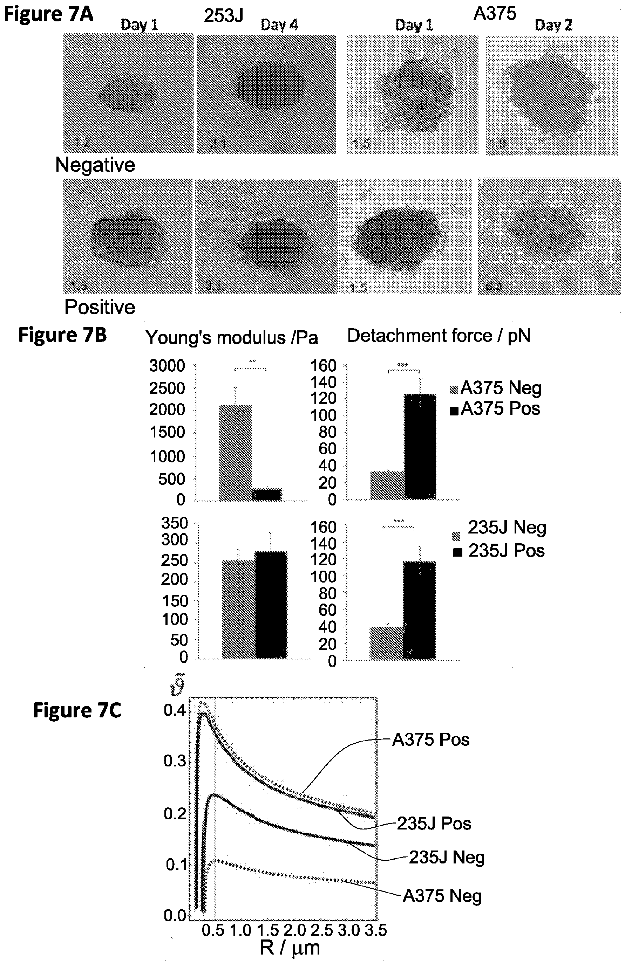

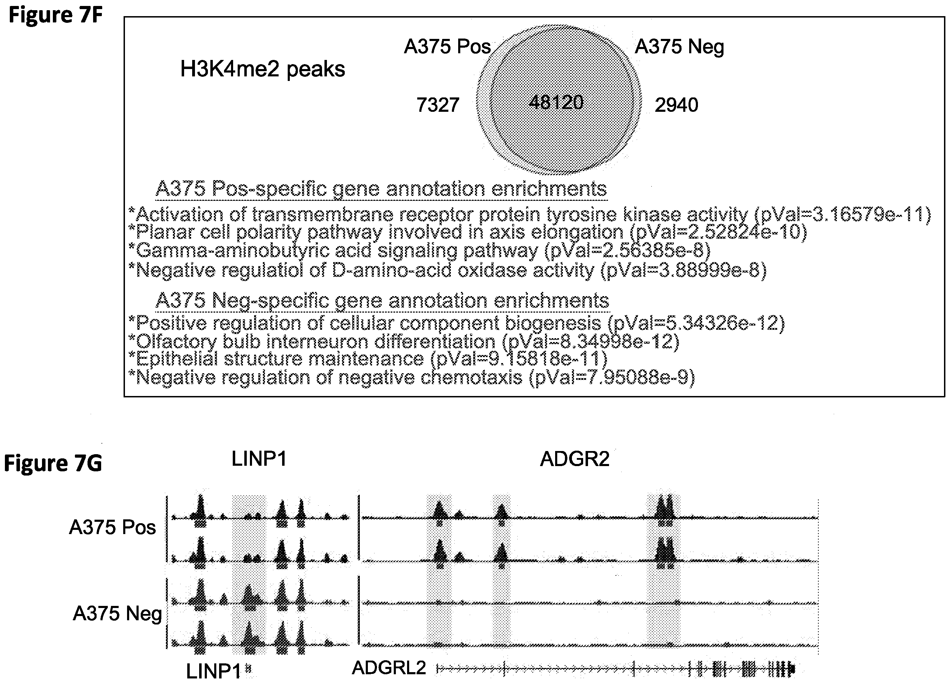

Figs. 6A-B demonstrate quantification of cell invasiveness in spheroids tests. Shown are original spheroid images as well as images formed by a MATLAB code for calculating spheroid perimeter and area. The compactness of the spheroids was calculated from the perimeter and area and marked in red. **(A)** PC3M-P vs PC3M-LN4 as appears in Figure 3D of the main test. **(B)** A comparison between Negative and positive sorting of both 253J and A375, as appears in **Fig. 7A****.**
   Using image analysis the invasiveness of cells was assessed from the outline shape of the spheroid image. The less smooth and spherical the spheroid was, and with more sprouting, the more invasive the cells were. This was measured by calculating the compactness of the spheroid defined as the dimensionless ratio between spheroid perimeter and area: $\frac{{perimeter}^{2}}{4 π ⋅ area}$. For a perfect circle the compactness equals 1, where the higher is the spheroid compactness the more invasive are the cells. The spheroid compactness was calculated using an image analysis MATLAB code which measured shape perimeter and area. The background of images was first cleaned using Photoshop for higher accuracy of the MATLAB code.
Figs. 7A-G depict TrC in cells that were sorted based on their uptake capacity. The cells with higher phagocytic capacity (positive cells) were more invasive and more deformable than the negative cells **(A-C). (A)** Spatial invasion assay with compactness values using spheroids of positive and negative A375-P and 253J cells showed that positive cells were more migratory and invasive than negative cells in collagen. **(B)** AFM results revealed that both of the positive subtypes were more adhesive than their negative counterparts. The A375-P positive cells were also more elastic than the negative ones. **(C)** The AFM results were used as parameters in the physical model. The stable wrapping angle was found to be larger in the positive cells compared with the negative cells (showing a higher effect in A375-P than in 253J, in agreement with the experimental results). The differences between positive and negative cells were further examined in bioinformatics assays **(D-E). (D)** GSEA plots showing up-regulation of cytoskeletal protein binding and EMT transition in A375-P and 253J cells. **(E)** Protein-protein interaction networks of genes are shown. Nodes represent proteins and an edge connects proteins that interact, where the size of a node is proportional to the number of proteins that interact with it. Light blue: 253J; Pink: A375; Mixed light blue and pink: nodes that contributed to the enrichment signal in both cell lines. **(F)** Venn diagram of H3K4me3 ChIP-seq peaks in A375 positive and negative cells. Overall we have identified 58,387 peaks, of which 48,120 peaks (82%) are shared among the two conditions. Conversely, 7327 peaks (13%) are unique to A375 positive cells, and 2,940 peaks (5%) to A375 Neg. Analysis of gene annotation shows enrichment for EMT-related terms among genes associated with the A375 Pos peaks, and maintenance of epithelial morphology-related terms among the A375 Neg-related genes. **(G)** Examples for A375 Pos and Neg differential peaks. An A375 Neg-specific H3K4me2 peak (left, highlighted box) is shown near the LINP1 promoter. Conversely, several A375 Pos-specific H3K4me2 ChIP-seq peaks were identified at the ADGRL2 gene (right, highlighted boxes). Both cases are in accordance with differential gene expression levels for those genes, as discussed above.

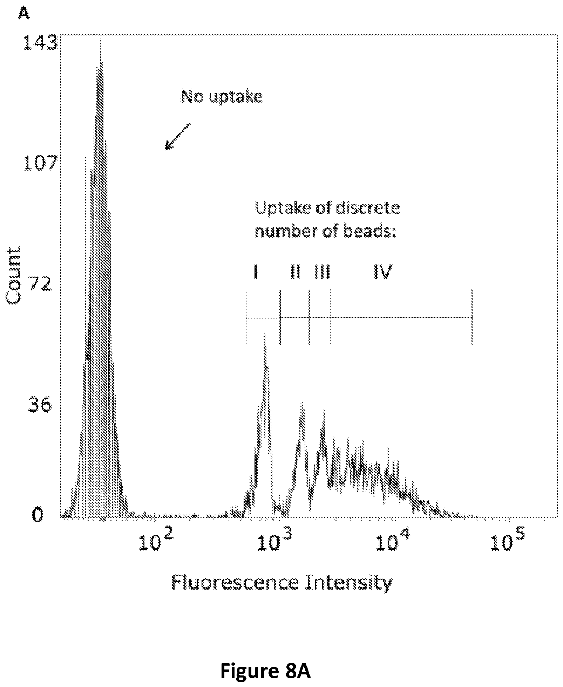

Figs. 8A-B show separate peaks of the FACS diagram corresponded to discrete number 2.4 µm beads inside the cells, allowing for accurate FACS sorter cell separation. (A) FACS fluorescence distribution of 253J cells 24 h incubation with 2.4 µm fluorescently labelled purple beads. The diagram shows well defined separated peaks. In the control experiments, a sorting procedure was done by separating cells based on their fluorescence intensity according to the different peaks. **(B)** Fluorescent microscope images of sorted cells originating from different peaks of the diagram show uniform subpopulations of cells, with an identical number of internalized beads in the vast majority of the cells. Due to the high internal fluorescence and the large size of the 2.4 µm beads, each peak corresponds to a well-defined number of cell-internalized beads, labeled (I-IV) in both panels A and B. The first peak on the left is made of cells that did not take any particles. The second to fourth peaks correspond to cells that internalized one particle, two particles, or three particles, respectively. The fifth peak corresponds to cells with four or more beads in each cell. Based on this, we used a FACS sorted scheme to separate precisely between positive and negative sub-populations as described in the main text.

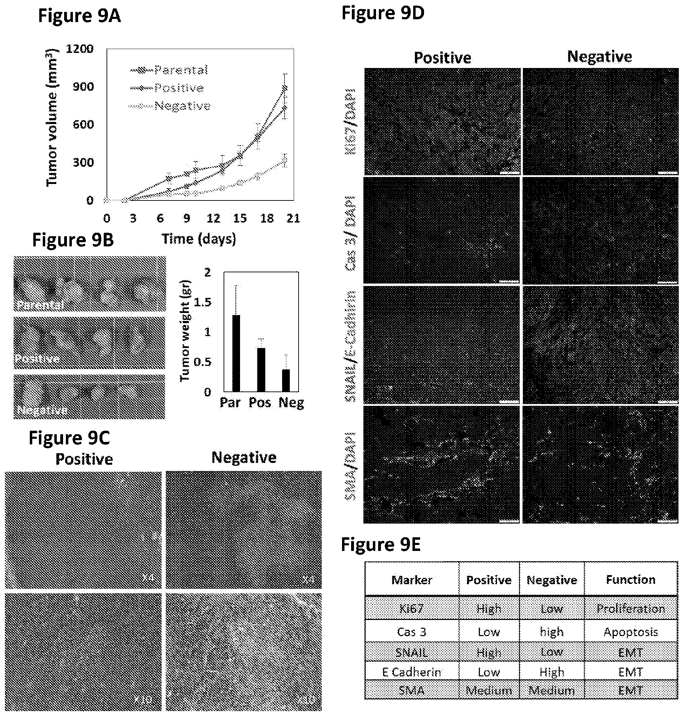

Figs. 9A-E demonstrate TrC in uptake based sorted cells was validated in a Xenograft study. Parental, positive and negative A375-P cells were injected s.c. to Athymic nude mice. **(A)** Induced tumors were measured every other, day from day nine following the injection. The negative tumors were notably smaller in volume than the positive and the parental ones. **(B)** Extracted tumor tissues from the negative group were significantly smaller compared with the positive and the parental tumors, as demonstrated visually and by weight measurements. Square scale = 1 cm. **(C)** H&E staining of the extracted tumors showed dense and highly proliferative tissues of the positive tumors and in contrast large areas of necrosis in negative ones **(D)** Immunohistochemical staining for evaluating proliferation (anti-Ki67 in green), apoptosis (anti-Cleaved Caspase-3 in red) and EMT (anti-SNAIL/SLUG in red vs. anti-E-Cadherin in green, as well as SMA in green). Positive tissues showed higher level of proliferation (ki67 staining) while negative tissues showed a higher level of apoptosis (Cleaved Caspase-3). SNAIL/SLUG vs E-Cadherin show enhanced EMT in the positive tumors compared to the negative ones, while SMA values were similar. Bar= 100 µm. **(E)** A qualitative summary of histological immunohistological analysis as determined visually from captured images. Grading is based on positive stained areas in a given tissue.
**Fig. 10** provides an ImageStream analysis of 0.8 µm particles by Aspc1 cells plated on PAA surfaces of varying rigidity. In this method, each cell that flows in the cytometry experiment is imaged and a detailed image analysis is done, where particles are defined and counted.

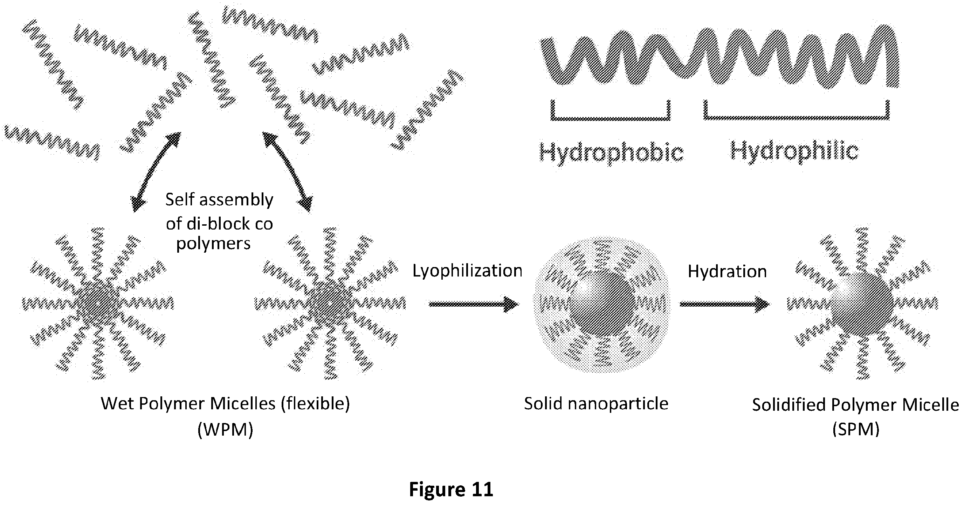

**Fig. 11** provides an illustration of polymeric micelles WPM and SPM fabrication. Di-block co polymers of PEG-PLA undergo self-assembly in aqueous media and form Wet Polymer Micelles (WPMs). Solid nanoparticles are formed by an additional step of lyophilization. Dry particles are hydrated once re-introduced into aqueous solution obtaining suspended Solidified Polymer Micelles (SPMs).

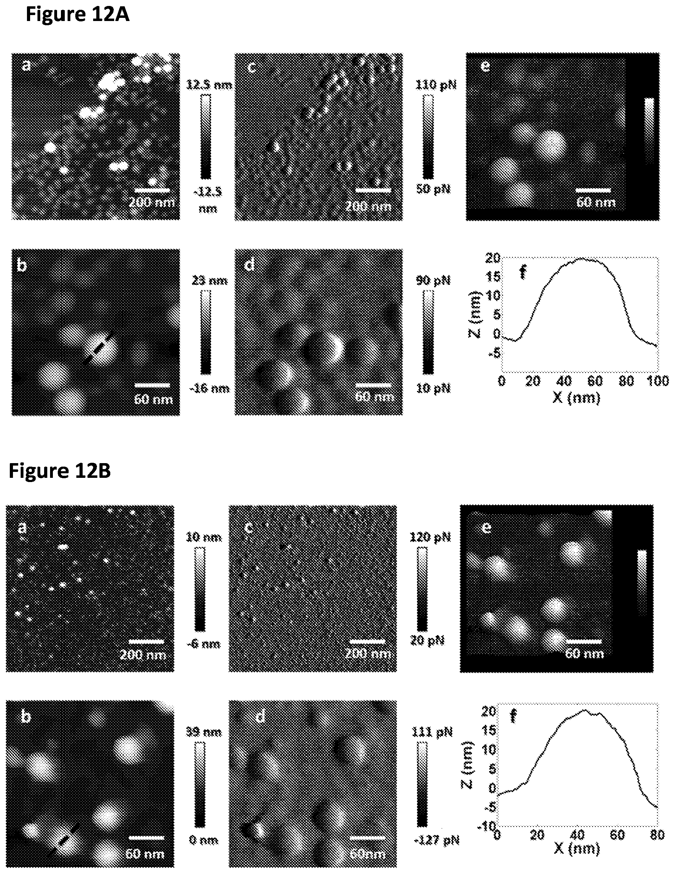

Figs. 12A-B provide micelle characterization using AFM. Images of the WPM **(A)** and SPM **(B)** in Height (a,b) and PeakForce modes (c,d). A 3D surface representation of (b) is shown. (f) shows a cross section profile along the dotted line marked in (b).

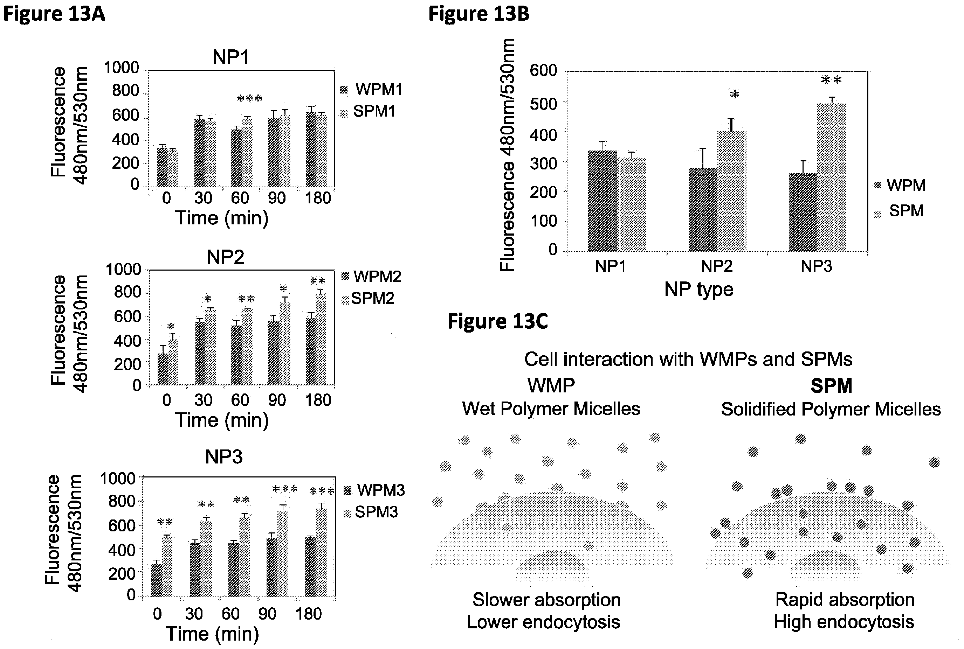

Figs. 13A-C show cell interaction with WPMs and SPMs. Cells interaction with NPs was tested by endocytosis into cancer cells. A375 cells were seeded in a 24 well plate (4×10⁴ cells/well). The next day equivalent quantities of fluorescent WPMs and SPMs were added to each well and washed twice with PBS after fixed time points. Florescence was measured by a plate reader with 480nm/530nm (Ex/Em). **(A)** NP1, NP2 and NP3 uptake by A375 cells. (**B**) Comparison of uptake in time 0. (**C)** Illustration of cell interaction with WPMs and SPMs.

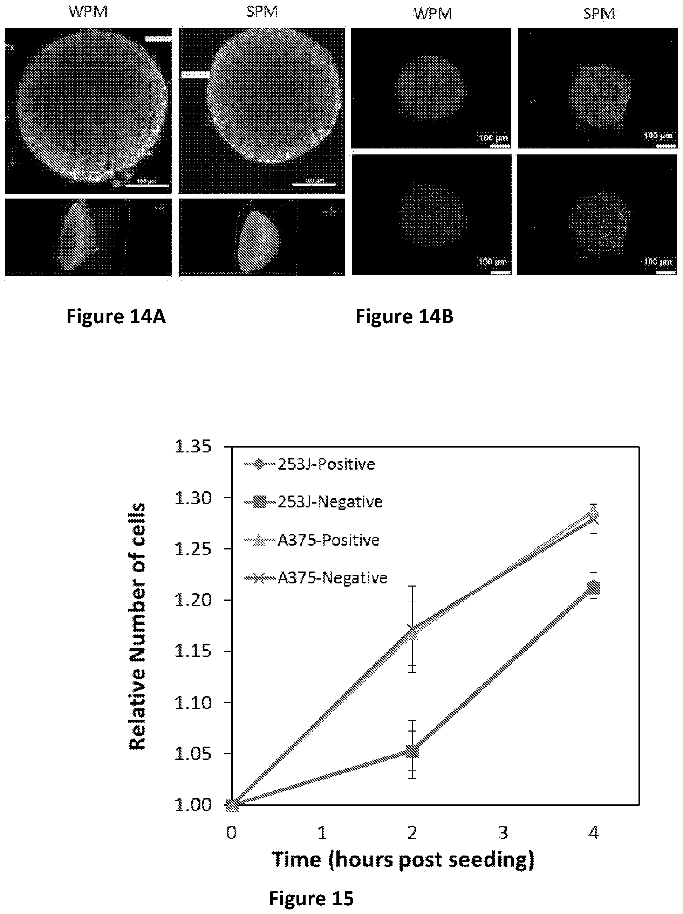

Figs. 14A-B depict NPs penetration into 3D tumor spheroids. BxPC3 cells were stained with DID and then seeded in a 35 well mold made from agarose (10,000 cells per well). The following day, spheroids were formed and incubated with WPMs and SPMs **A.** Spheroids incubated overnight with NP3 were imaged by 2-photon microscope (Nikon A1 MP Multi-Photon). **B**. Spheroid cells were stained with DID and incubated for 3 hours with NP3. Spheroids were then frozen in OCT, sectioned and imaged by fluorescence microscopy.
**Fig. 15** shows cycle time of cells was not affected by the FACS sorting procedure. This important control ensured that differences in cell behavior (e.g., uptake pattern) between positive and negative sub-populations did not result from differences in cell cycle time. Sorted cells from the 4th cycle of sorting (20,000 cells/well A375 cells and 30,000 cells/well 253J cells) were seeded in triplicates in a 96 well-plates and their proliferation rate was monitored for 96 h using cell counting using an Invitrogen Countess automated counter.

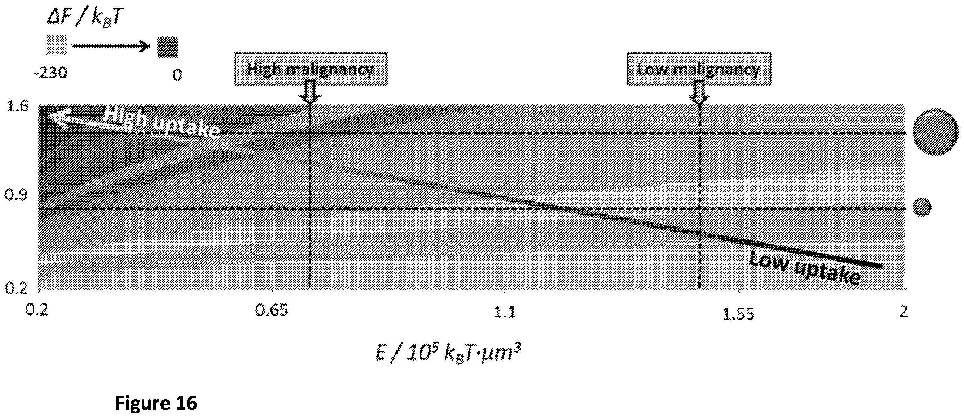

**Fig. 16** depicts physical theory of Mechanical Targeting. A surface plot of the change in free energy in particle engulfment by cells as a function of the cell elastic moduli, which is a biomarker of malignancy, and particle size. The fingerprint uptake pattern (e.g., the cross points of the dashed lines).

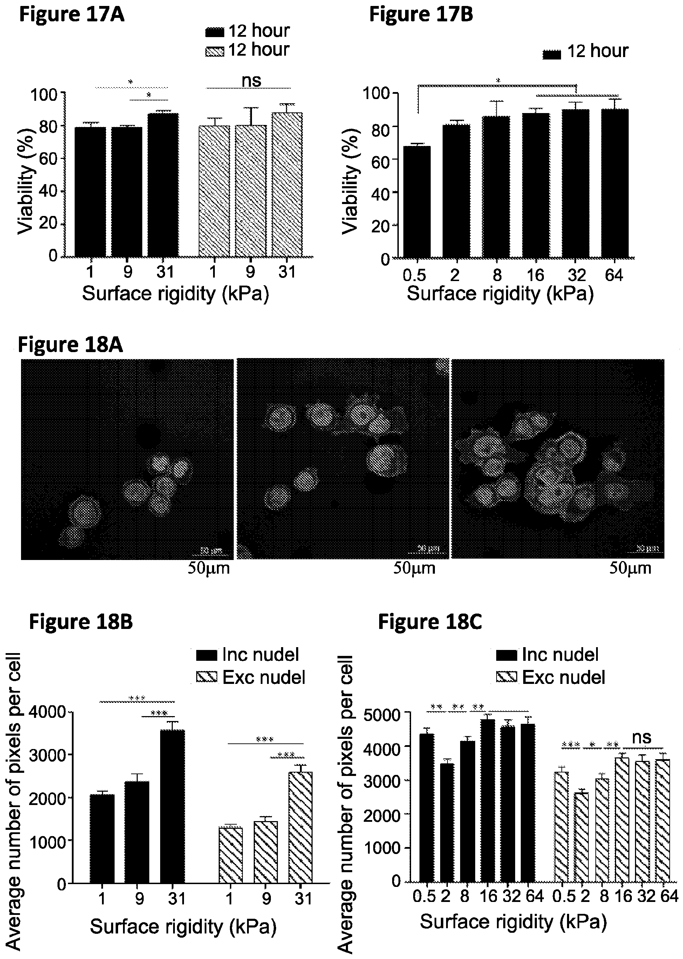

Figs. 17A-B show substrate stiffness affects AsPC-1 cells viability. (A) Viability assay of AsPC-1 cells using MTT, after 12 and 96 hours of incubation on fabricated surfaces of ranging stiffnesses (1, 9 and 31 kPa), investigating effect of surface stiffness on cells' viability. n=6. *p < 0.05, normalized to viability of non-treated control cells with the same incubation time. Viability of AsPC-1 cells grown on commercial surfaces for 12 hours was evaluated using MTT **(B)** Results are presented as mean ± SEM normalized to viability of cells grown on surfaces of 64 kPa. *p < 0.05, **p < 0.01, ***p < 0.001, ns, not significant.
Figs. 18A-C show cell spreading is higher on more stiff surfaces. (**A**) Representative fluorescent image of AsPC-1 cells cultured on fabricated polyacrylamide surfaces of varying stiffnesses for 12 hours, which indicates on their spreading when in contact with particles. Cells were stained with Phalloidin-iFluor 555, and DAPI. Scale bar: 50 µm. The exposed cell surface that is available to particle absorption was evaluated using image analysis of cells plated on fabricated (**B**) and commercial (**C**) substrates for 12 hours. The average size of cells was calculated including and excluding nuclei. Results are presented as mean ± SEM. *p < 0.05, **p < 0.01, ***p < 0.001, ns, not significant.

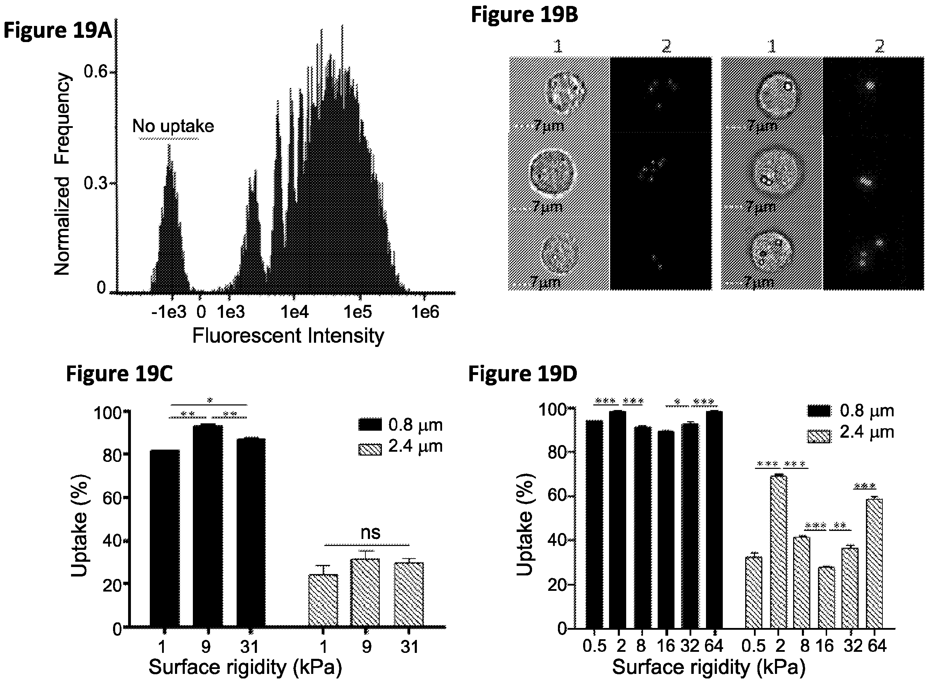

Figs. 19A-D Surface stiffness affects AsPC-1 cells' uptake capacity. (**A**) Bead uptake was evaluated by ImageStream using fluorescent channel intensity histograms. The X axis represents the integrated intensity of the fluorescent channel of beads in the object image. **(B)** Representative images of cells captured using ImageStream; 1-brightfield images of cells, 2- fluorescent images of beads. Scale bar: 7 µm. **(C, D)** ImageStream uptake analysis was performed following addition of 0.8 and 2.4 µm fluorescent spherical beads, after hours of incubation with AsPC-1 cells seeded on fabricated **(C)** and commercial **(D)** surfaces of varying stiffnesses. The Y axis represents the percent of cells from the total population that internalized any number of beads. n=3. P values presented in the graph show a significant non-monotonic dependence of the surface rigidity on the cells' uptake capacity. Results are presented as mean ± SEM. *p < 0.05, **p < 0.01, ***p < 0.001.

### DETAILED DESCRIPTION OF EMBODIMENTS

### General

The methodology steps are summarized in **Fig. 1****.** In general, the methodology involved rough fractioning between normal and cancer cells, analysis of the cancer cells fingerprint and providing a diagnostic score C. The score captures information on (a) the percent of malignant cells in the population and (b) the detailed mechanical signature of the cancer cells, accounting for cross-correlation in the uptake patterns of several reporting particles as detailed below.

Initially, biopsy cells undergo FACS streamer or other types of uptake experiments with a set of reporting particles. The set is determined prior to the clinical applications by a comprehensive study of uptake in several types of cells of known malignancy with a large matrix of reporting particles. This is followed by large data analysis and supported by physical knowledge provided by a theoretical model, as detailed below.

An initial analysis determines the fraction of normal vs. cancer cells from the sample population. This can be done by defining a general uptake threshold. A preliminary definition would be that normal cells are identified as those that do not uptake any particles. This analysis provides the fraction of cancer cells parameter 0 < *A* < 1 (in the illustrated example = 0.8) .

In the second step, the fingerprint pattern of the cancer cells is identified. This is done by building a multidimensional uptake histogram (illustrated in the **Fig. 1** as one dimensional for clarity). Each point of the histogram reports on the fraction of cancer cells which internalized specified number of the particles. For example, 0.24 of the cells internalized 0-2 particles of type A, 7-8 particles of type B and 3-4 particles of type C. Each one of these particle populations, indexed here by *i*, has a weight which reflects its relevance in affecting the malignancy, *Q(i).* The matrix of weights is determined in a pre-research. Particles that require large cell deformation for their engulfment are predicted to lead to high levels of *Q.* For example, suppose that particle A is difficult to "swallow" and B and C are internalized easily, than the weight of internalizing 7-8 A particles, 3-4 B particles and 5-6 C particles would be larger than the weight of internalizing 0-2 A particles and 12-14 B and C particles, and so forth.

In the last step, a diagnostic score is provided, which integrates the information at hand, as explained above. This may be calculated using the following: $C = A {\sum }_{i} Q \left(i\right) ⋅ Uptake \left(i\right)$ where *Q*(*i*) and *A* are normalized so that the scoring obeys 0 < C < 1. Such a scoring provides a single parameter that captures a large data set. In a multiparameter case, A for example may be the first value and Σ*ᵢ Q(i)* · *Uptake(i)* may be the second.

### Results

### A. Rigid particles: Triangular correlation between cell deformability, phagocytic like capacity, and cancer aggressiveness.

### Malignant cells uptake inert particles more readily in a size-dependent manner

We examined if cancer cells have enhanced abilities of particle uptake as compared to normal cells from the same tissue-origin (Figs. 2A-C). Skin cells from a primary cancerous cell line (melanoma, A375-P) were compared to transformed skin cells originated from normal cells (Hacat, keratinocyte cell line). Both cells were incubated with fluorescent polystyrene particles, ranging from hundredths to several microns in size, and uptake was evaluated by florescence measurements using flow cytometry, fluorescence microplate reading (Figs. 2A-B and by microscopy **(****Fig. 2C****).** After overnight incubation, we consistently found, using all three methods, that the cancer cells had dramatically taken more particles than the normal cells. Vanishing uptake of small particles below 0.5 µm was observed (explained below).

In order to determine the relevant particle size for studying the TrC, eight different cell lines were tested from skin, prostate, bladder and kidney cancers, which differed in their metastatic potential (see Methods). Experimental and theoretical results in **Fig. 3** show a non-monotonic dependence where bead uptake increases with particle size, reaching a maximal value for 2.4 µm beads, and then declines. This was observed using FACS, plate reader **(****Fig. 3A****)** and confocal imaging **(****Fig. 3B****),** which also verified particle penetration into cells. The non-monotonic dependence was predicted by a physical theory as illustrated in **Fig. 3C****.** Engulfment and uptake of particles by cells require a sufficiently large area of cell-particle adhesion to reduce the free energy, but minimal cell morphological changes as the latter increases the free energy. Hence, intermediate sized particles are optimal here for cell entrance, having a wide enough contact area with cells and requiring small cell deformation during their engulfment.

The physical theory proposed here is a combination of contact mechanics principles for 3D cell deformations upon contact with a particle, as well as a 2D description of cell membrane morphology, providing the following change in free energy: $Δ F \left(ϑ\right) = - 2 {πR}^{2} Δ γ \left(1-cosϑ\right) + 3 {ER}^{3} {\left(1-cosϑ\right)}^{2.5} + 4 πκsinϑ ⋅ ϑ$

*ϑ* is the reaction coordinate describing particle wrapping by the cell membrane. The particle is modeled as a rigid sphere of radius *R.* The first two terms describe the adhesion and 3D elastic cell deformation according to the Derjaguin-Muller-Toporov (DMT) model. Δ*γ*, the work of adhesion per unit area, and *E,* the effective Young's modulus of the cell, are calculated here per cell type from our AFM results **(Table 1).**

**Table 1: Cells parameters calculated from AFM measurements, as well as the critical radius for particle uptake based on the physical model results. Cell specific parameters, E and Δγ, appear in the left and middle columns of the table, respectively, in different cell lines, including the positive and negative sub-populations of FACS sorted cells. These parameters were analyzed based on AFM data using a DMT model (see Methods). The values of E and Δγ were used as input parameters in our physical model. Right column: values of the critical radius for particle uptake calculated using the equation derived from our physical model:**

| | *E* (kT/µm³) | Δ*γ* (kT/µm²) | *R_{c}* (µm) |
|---|---|---|---|
| PC3M-LN4 | 77020.2 | 461.8 | 0.20 |
| PC3M-P | 45835.3 | 869.1 | 0.14 |
| A375 Pos | 64690.0 | 789.1 | 0.15 |
| A375 Neg | 512970.0 | 208.3 | 0.29 |
| 253J Pos | 67681.3 | 729.4 | 0.16 |
| 253J Neg | 62007.8 | 248.0 | 0.27 |

${R}_{c} ≡ \sqrt{\frac{4 κ}{Δ γ}} ,$ where *κ*=4.5*kBT* is the membrane bending modulus.

The third term reflects the elastic membrane curvature with bending modulus *κ* = *4.5k_{B}T* in thermal energy units, where *k_{B}* is the Boltzmann constant and *T* the temperature. The model derivation and assumptions can be found in Online Methods.

Importantly, the function Δ*F*(*ϑ*) has a minimum only when the particles are larger than a critical value: $R > {R}_{c} where {R}_{c} ≡ \sqrt{\frac{4 κ}{Δ γ}}$

For small beads below the critical radius *R_{c}*, Δ*F* is a monotonically increasing function **(****Fig. 3D****)** and it is not expected that particles will be uptaken by cells, as indeed observed in our experimental results. Given cell type, *R_{c}* values were measured here using AFM, and are presented in **Table 1**. We found an excellent match with experiments in predicting realistic values of *R_{c}* below which uptake capacity was vanishing. From **Eq. 1** we can derive the stable (equilibrium) wrapping angle, *ϑ̋*; the higher *ϑ̋* is, the more likely it is to have full engulfment and uptake. For intermediate values of *R, ϑ̋* is maximal **(****Fig**. **3E****)** in agreement with the experimental findings (Figs. 3A-B) due to the energetic considerations mentioned above. From the dependence on *E,* the stiffer the cell the less bead uptake is probable, as indeed found in our experiments (see next section). The model geometry is sketched in **Fig. 3F****.**

### TrC in parental vs metastatic cancer cells

We compared cells with varying malignancy that originated from either a primary site of prostate cancer (PC3M-P) or a metastatic subpopulation of these cells (PC3M-LN4). A comparison of the three factors - particle uptake, mechanical deformability, and malignant potential between these cells - is presented in **Fig. 4**. Particle uptake was found to be higher in PC3M-LN4 cells as measured by FACS **(****Fig. 4A****)** and confocal microscopy **(****Fig. 4B****)** and as predicted by our model **(****Fig. 4C****).** In the physical model, we used specific cell parameters from **Table 1** and found that the free energy is lower and that *ϑ̋* is larger for the more metastatic cells, predicting a higher uptake as indeed observed by the FACS and the confocal microscope. The highly phagocytic-like PC3M-LN4 cells were also found to be more invasive. **Fig. 4D** shows an *ex vivo* 3D multicellular spheroid formation assay in which the PC3M-LN4 cells were clearly more invasive in collagen as compared to PC3M-P cells. This was reflected in spheroid contour which was rougher and with more detaching cells. **Fig. 4E** presents AFM results. The Young's modulus of the PC3M-LN4 cells was smaller than that of PC3M-P cells and their maximal adhesion force was larger. This indicates on higher mechanical deformability of PC3M-LN4 compared to PC3M-P cells, as the PC3M-LN4 cells were more compliance to shape changes induced by indentation or retraction of the AFM probe.

### Particle uptake based cell sorting differentiates cells according to their cancer aggressiveness

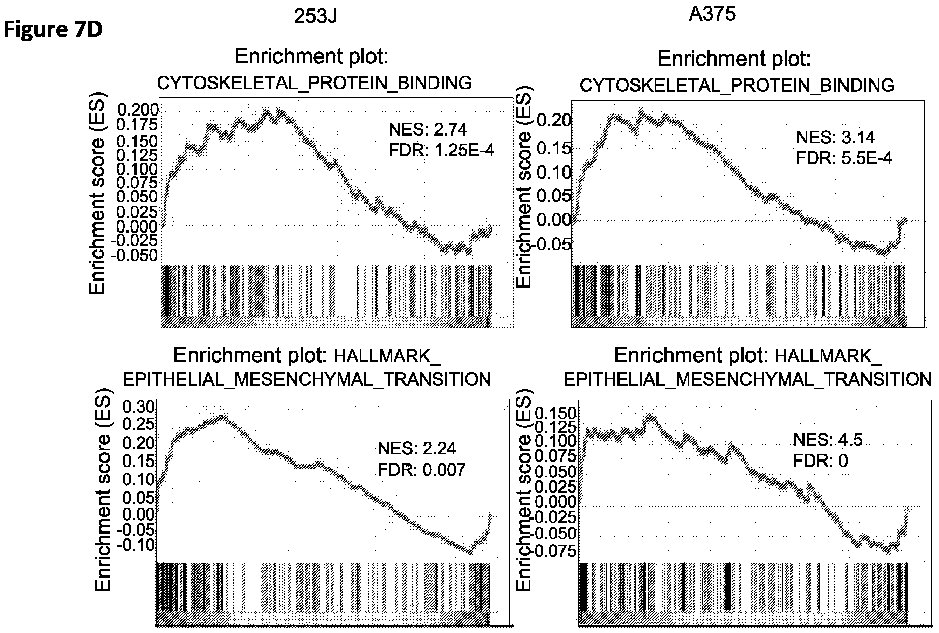

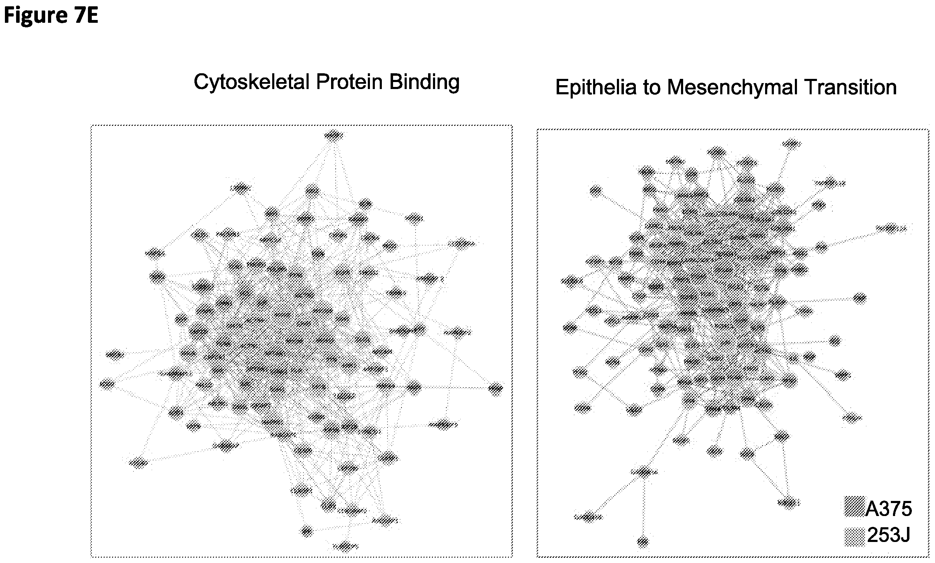

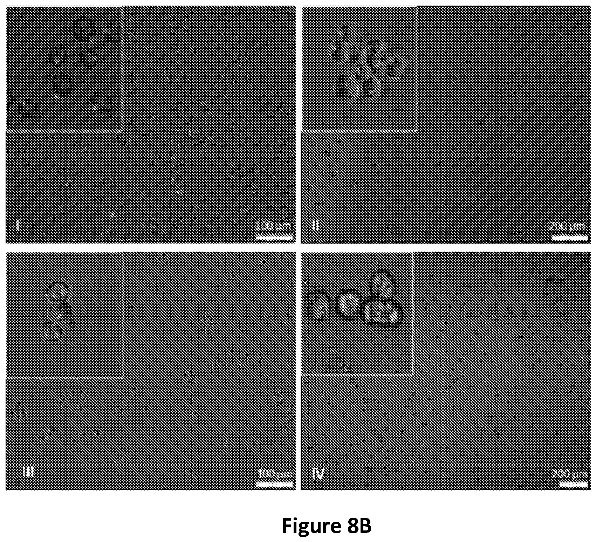

We found an innate heterogeneity in uptake capacity among given cancer cell populations (**Fig. 5A**). Hence, we sorted cancer cells from the same cancer origin by their "phagocytic" potential, aiming to determine whether such sorting can also subgroup cells according to their malignancy potential and their deformability. In our scheme **(****Fig. 5B****),** a FACS sorter was used for separating cells that did not take any particles ("negative") from cells that had taken up any number of particles ("positive"). We used 2.4 µm beads, for which separate peaks of the FACS diagram corresponded to discrete number of cell inserted beads **(****Fig. 6****).** This allowed us to control the number of beads taken by cells in the FACS sorting procedure. We started with two types of primary cancer cells: bladder (253J) and melanoma (A375-P). Six cycles of FACS sorting were made yielding very efficient enrichment patterns **(****Fig. 5C****),** indicating that uptake potential is a preserved characterization. The enrichment cycles resulted in four subpopulations: 253J positive, 253J negative, A375-P positive and A375-P negative. Strikingly, the positive cells (both in 253J and A375-P) showed a more invasive phenotype as compared to the negative ones in a spheroid invasion test **(****Fig. 7A****).** Importantly, we found that proliferation rates in positive and negative sub-populations were identical **(****Fig. 8****)** and did not cause differences in cell behavior. AFM measurements **(****Fig. 7B****)** confirmed TrC, showing that both of the positive subtypes were more adhesive than their negative counterparts. The A375-P positive cells were also more elastic than the negative ones. The AFM results were used as input parameters in the physical model **(****Fig. 7C****).** In both cell pairs the model predicted a higher extent of engulfment in positive vs negative cells, leading to higher uptake in those cells. To examine the preservation of the "phagocytic" phenotype, we performed RNA sequencing of the four subpopulations. GSEA was used to analyze gene set enrichment on the hallmark gene set collection of the molecular signature database (mSigDB). In both cell lines uptake-positive enriched cells displayed upregulation in the transcription of EMT and cytoskeleton-related gene sets **(****Fig. 7D****).** Top cytoskeleton-related upregulated gene sets included GO_actin_cytoskeleton, GO_cytoskeletal_protein_binding, G0_actin_filament_based_process, KEGG_regulation_of_actin_cytoskeleton, and GO_structural_constituent_of_cytoskeleton. **Fig. 7E** shows protein-protein interaction networks of the genes that contributed to the upregulation.

For a stronger level of validation of the malignant potential of the positive sub-populations, we performed an *in vivo* experiment. Three groups of nude mice were injected subcutaneously with cells originating from our sorting experiments: i) original A375-P cells, ii) positive A375-P cells, and iii) negative A375-P cells. Tumor growth was monitored over 20 days in C57/B1 mice. A significant (p=0.033) difference in tumor volume was obtained after nine days with negative cells having 52% and 64% smaller tumors compared to positive or parental cells, respectively **(****Fig. 9A****).** Tumor images are presented in **Fig. 9B****.** It was expected that there would be a large divergence of the negative population and similarity between positive and parental cells since the negative cells were formed by specifically isolating "non-phagocytic" cells from the rest of the population. As shown in **Fig. 9C****,** histological examination of hematoxylin and eosin (H&E) stained tissue sections revealed that positive cells induced dense and highly proliferative tumor tissues while negative ones formed tissues with large areas of dead cells. To investigate whether the necrotic area in the negative cell-injected tissue resulted from cell apoptosis or from excessive proliferation, we performed immunohistological analysis using ki67 as a marker for proliferation and Cleaved-Caspase 3 to label apoptotic cells (**Fig**. **9D****, top**). Accordingly, we suggest that the positive cell-originated cancers were more proliferative and showed reduced apoptosis as compared to the negative ones. Moreover, three EMT related markers, Snail, E-cadherin and SMA (**Fig. 9D****, bottom),** were tested to assess their level in the tumor tissues. The results revealed that Snail had higher levels in the positive tissues while E-Cadherin was lower, and that SMA levels were shown to be similar. The histological findings are summarized in **Fig. 9E****.**

### Particle uptake depends on the mechanical properties of the environment.

Mechanical properties of the environment of the cells can affect the capacity of cell interaction with particles. **Fig. 10** shows an analysis done over tens thousands of cells using FACS ImageStream. In this method, each cell that flows in the cytometry experiment is imaged and a detailed image analysis is done, where particles are defined and counted. Interestingly, a non-monotonic dependence of the interaction on substrate rigidity was found, where minimal uptake was observed on surfaces of 4 kPa, and higher values for less (1 kPa) and more (16 kPa) rigid surfaces. This result can be explained in the duality of the role of surface stiffness. When the surfaces are more rigid, on the one hand cells spread to larger, increasing the available area for interaction with particles. On the other hand, cells are stiffer on rigid substrates thus the energetic penalty upon interaction with particles is larger. These contradicting effects result in non-monotonic dependence observed in **Fig. 10****.**

### Discussion

Growing knowledge about the high complexity of tumor cell biology has made it clear that an integrative approach is required in cancer research, including cell mechanics aspects. Mechanical deformability (measured here in terms of elasticity and non-specific adhesiveness) was found here to be the mechanistic link that correlates cell uptake features with cancer aggressiveness. Correlations between cell deformability and cancer potential were previously studied. Various physical examinations were performed comparing the flexibility of normal and cancer cells with increasing malignancy. In numerous types of cancer it was found that cell elasticity could be used as a biomarker for malignancy and metastatic potential. Some studies found that a dramatic reduction of cell stiffness resulted in lower invasiveness potential, probably due to the difficulty in sensing and applying force. While single cancer cells *in vitro* show enhanced elasticity, 3D tumor tissues were very stiff as compared with normal ones. This duality highlights the significant ability of cancer cells to adjust mechanically to different conditions such as the force exerted by the ECM. It is well-known that cell adhesiveness plays a major role in cancer progression. Turnover of specific cell adhesion molecules is required for enhanced dynamics of both cell-cell and cell-ECM adhesions during tumor growth and the spread of metastases. While most previous data focused on the relation between specific adhesions and malignancy, importantly we found here that non-specific adhesions were also correlated with cancer potential. Namely we found that cell deformability, in terms of elasticity and non-specific adhesiveness, is the mechanistic link between cancer aggressiveness and cell uptake capacity.

To date, information is sparse regarding the role of cell mechanics in particle-loading by cancer cells. A recent work by Ma et al. showed that drug resistant soft tumor-repopulating cells loaded cell-derived microparticles that contained drugs more efficiently than parental tumor cells that were stiffer and originally less resistant to drugs. Plating cells on surfaces of varying stiffness or topography is an effective tool for controlling the cell mechanical state. Using such plating methods, higher particle uptake by more deformable cancer cells was found, however some different results were observed as well, probably due to opposing effects of cell spreading. On the one hand, larger spreading limits uptake by inducing high cell stiffness, but on the other hand, it provides larger available area for particle absorbance and penetration. Cancer cells often have enhanced abilities to internalize large micro-objects, a property commonly referred to as a phagocytic ability. Evidence of cancer cell cannibalism also implies on advanced capacities of internalizing large objects. In a work by Chandrasoma published in 1980, the author examined cells from a primary tumor of a patient with endometrial adenoacanthoma as well as from skin metastasis of the same patient. In the primary tumor, two cell populations were recognized; only one of them phagocytosed polymorphonuclear leucocytes. However, all metastasis cells phagocytosed polymorphs. It was suggested that the phagocytic abilities of primary tumor cells were related to their potential to form metastases. Other reported studies outlined a phagocytic ability of cancer and metastases cells, however direct comprehensive evidence of the aggressiveness - uptake link was not clear. Here, we found that cancer cells with higher metastatic potential had elevated capacities of particle uptake and that cell populations that were sorted based on increased phagocytic properties were more aggressive both *in vivo* and in artificial 3D tumors. This provided a direct proof that the phagocytozing cells were more aggressive and vice versa.

Dramatic differences between particle uptake by keratinocyte cell line (originated from normal tissue) and cancer skin cells were found here, supporting the TrC. Moreover, we found that primary prostate cancer cells (PC3M-P) internalized particles five-fold less than a metastatic subpopulation of these cells (PC3M-LN4). The cells metastatic potential was validated using spheroid invasion tests showing higher invasiveness of the PC3M-LN4 cells, which were found to be also more elastic and adhesive in the AFM experiments. The AFM measurements were used as input parameters in our physical coarse-grained model, which qualitatively predicted uptake patterns of the primary and metastatic cells using basic energetic arguments. The free energy terms of particle engulfment by cells include the interplay between the reduction of energy caused by cell-particle adhesion and an increase in energy resulting from cell deformation upon particle wrapping. We found here that cells of higher malignant and metastatic potential were more elastic and adhesive. Thus, the energetic penalty of their deformation is smaller and the gain in adhesion energy is larger, resulting in higher uptake capacity.

The size of particles was found to affect dramatically the extent of uptake. A non-monotonic dependence of uptake on particle size was found, with a maximum uptake for 2.4 µm beads. Other size dependencies reported in the literature, could have resulted from varying values of the adhesiveness and elasticity of cells, or from biological factors such as the membrane surface brush or specific biochemical pathways such as clathrin or caveolin-mediated internalization and formation of lipid rafts. Here we tested beads of minimal cell affinity, thus adhesion energy was in-sufficient to enable particle internalization in the case of small particles due to the small cell-particle contact area. Increasing particle size widens the contact area and thus uptake becomes more favorable. However, a further increase in particle size requires massive cell deformation upon engulfment and the uptake is reduced, resulting in a non-monotonic dependence. Moreover, our model predicted the experimental observation of a critical radius, *R_{c}* = $\sqrt{\frac{4 κ}{Δ γ}}$, (specific for cell and particle type) below which particles do not penetrate cells.

A major challenge in cancer treatment results from internal heterogeneity and plasticity in terms of genetics, functionality, phenotypes, stem cell differentiation, microenvironment variability and others. It should be recognized that mechanical heterogeneity is significant as well, and that, ideally, all of these factors should be accounted for in precision treatments. We found a large variability in uptake capacity of cancer cells from the same population **(****Fig. 5A****),** raising two questions. Is uptake variability correlated with cancer potential and mechanical properties of individual cells? And are these characteristics inherited from generation to generation, and can therefore allocate potential targets for therapies? To address these questions, we sorted cells based on uptake capacities using six enrichment cycles of FACS sorting. Differences between the sorted cells were striking and indicated conservation of the Triangular- Correlation. The fact that sorting of cells according to their particle uptake resulted in populations that were clearly distinguished from each other in their cancer potential after mice injection *in vivo* and in artificial 3D tumors (spheroids), may have an enormous impact on future drug design and diagnostics. Our gene expression analysis supported the mechanical link between uptake and cancer potential, suggesting the differential pattern of upregulation in the transcription of EMT genes and genes related to cytoskeleton reorganization. Histologically we found that positive cell populations had more aggressive phenotypes, enhanced proliferation, and an increase in EMT protein, Snail and reduced E-Cadherin expression, suggesting an epithelial to mesenchymal transition.

Seeding cells on substrates of increasing rigidity raises both the spreading area and the stiffness of the cells. Therefore, changing the rigidity of the substrate can be used as a method for manipulating cells from the same origin into different mechanical states. **Fig. 10** shows that the increase of substrate rigidity results in a decrease and then an increase in the interaction with 0.8 µm particles. This is explained by the opposing effects of the increase in cell area and rigidity.

The significance of combining cell mechanics in the rational design of cancer therapies and diagnostics is clear. However, robust, accurate and simple mechanical tests for this aim are lacking. Importantly, a consequence of the TrC is that uptake measurement can be used as a reliable and efficient scheme of mechanical assessments in cancer. We anticipate that these findings will open up the possibility of practical procedures that use uptake tests as a routine in cancer diagnostics and in the rational design of selective therapies. Further development our findings would involves focusing on clinical scenarios that can benefit from our TrC insights related to cell-particle interactions, including ex-vivo diagnostics, or circulating cells. At the tissue level of-course additional effectors should be considered. Future research in this field can include investigations of different aspects of mechanical factors such as biochemical pathways and cytoskeleton remodeling. In addition, the efficacy of drug-loaded carriers can be examined for enhancing specificity based on mechanical arguments.

### Rigidity of polymer micelles affects interactions with tumor cells

Controlling the interaction of drug delivery systems (DDS) with tissues is critical for the success of therapies. Specifically in cancer, due to the high density of the tumors, tissue penetration of DDS is critical and may be challenging. Here we have compared the interaction of cancer cells in vitro and in three dimensional spheroids (ex vivo) with either Solidified Polymer Micelles (SPM) or Wet Polymer Micelles (WPM). SPM and WPM have identical molecular components and differ in their rigidity, where SPM are stiffer than WPM as explained in **Fig. 11****.**

We have used here 80 nm micelles of both types. **Fig. 12** shown that the solid particles interact more with A375 cells. This was predicted since the flexible particle can increase their contact area with the cell and there for their engulfment can require more massive cell deformation. This trend is also demonstrated in the time dependence imaging shown in **Fig. 13****.** Importantly, the same behavior was observed in 3D spheroids, where the interaction of the cells with SPM was higher than with WPM, as shown in **Fig. 14****.**

### Discussion

The results presented herein indicate of an important role of nanoparticle flexibility in cell uptake. Specifically, we have shown here that stiffer particles are internalized more into cancer cells compared to softer ones, in accordance with a previous study. Since the WPMs and SPMs have similar size and are made of the same building blocks, they have the same charge and are expected to have the same chemical interaction with the cells. These characteristics allow for isolated examination of the role played by particle rigidity. This principle may be valid to other drug delivery systems and may explain cellular internalization kinetics and the effect of solid nanoparticles such as poly lactic-co-glycolic acid (PLGA) or gold nanoparticles in comparison to performance of "wet" systems such as liposomes.

Since we see indication of differences in cellular uptake between SPM and WPM we hypothesize that such differences can be manifested also in multicellular structures. Therefore, we measured cellular uptake in a 3D cell model of tumor spheroids which better mimics the physiological behavior. Even though quantitative data is not presented here we clearly see differences in micelles uptake of BxPC3 spheroids.

Since many anti-cancer drugs exhibit poor penetration abilities into tumor tissue formulations that may improve drug penetration into tissues could have substantial implication in efficacy of treatments. Our findings indicate that mechanical cues have an important effect on the ability of nanoparticles to interact with cells. We found that stiffer polymer micelles nanoparticles have advantageous penetration abilities into tumor cells leading to higher uptake, also in multicellular entities. Using distinction of uptake abilities based on mechanical properties of micelles, NPs could be of an importance factor in designing efficient drug delivery systems for specific physiological conditions. Our finding may have a broader impact and relevance to other drug delivery systems, such as liposomes, emulsions and hydrogels and biodegradable particles.

### Materials and Methods

All institutional and national guidelines for the care and use of laboratory animals were followed and protocols were approved by the Hebrew University of Jerusalem Ein Kearem Medical School Institutional Animal Care and Use Committee.

### Cell culture

Human keratinocyte cell line, HaCaT cells (ThermoFisher) maintained in Dulbecco's modified Eagle's medium (DMEM, ThermoFisher) supplemented with 10% (V/V) fetal bovine serum and 1% antibiotics (10,000 µg/ml streptomycin and 10,000 units/ml penicillin) at 37°C with 5% CO₂. A375-p (primary human melanoma cells), A375-SM (metastatic human melanoma cells), PC3M-P (primary prostate cancer cells), PC3M-LN4 (metastatic prostate cancer cells), 253J (primary human bladder cancer cells), 253J-B5 (metastatic human bladder cancer cells), SN12 (human renal primary cancer cells), and SN12-C SN12 (human renal metastatic cancer cells) were obtained by the Bielenberg laboratory at the Boston Children's hospital, USA, and maintained as previously reported. Cells were serially passaged at 70-80% confluence and then experiments were conducted with subconfluent cells. All cell-lines were Mycoplasma-free (EZ-PCR Mycoplasma Test Kit Biological Industries, catalog number 2070020).

### Materials and reagents

Unless otherwise stated reagents were purchased form Sigma-Aldrich. Fluorescently labelled polystyrene spherical particles were purchased from Spherotech Inc. (USA). In most cases, purple fluorescence (Ex. 488nm, Em. 545/60nm) was used except for the 6 µm particles in **Fig. 2B** that were pink (Ex 488nm, Em. 615/30nm). Antibodies for immunostaining were: anti-Ki67 (1:100, Abcam, # ab15580) for proliferation, anti-Cleaved Caspase-3 (1:100; Cell Signaling, #MAB835) for detecting apoptosis, and markers for EMT: anti-SNAIL/SLUG (1:100, #ab180714), anti-E-Cadherin (1:100, Abcam, #ab15148) and anti-smooth muscle actin (SMA, 1:100, Abcam, #ab5694). Cells were blocked with 3% rabbit serum

### Measurement of particle uptake of by cells

To measure the extent of particle internalization into cells, three methods were used: fluorescence-activated cell sorter (FACS) analysis, spectrometry microplate analysis and imaging. Assessments of uptake after 24 h incubation with fluorescently labeled polystyrene particles were performed. Control experiments were done with 5 min incubation, ensuring low particle - cell affinity that does not depend on particle size **(****Fig. 15****).**

For FACS analysis, cells were seeded in six well plates for four days after O/N starvation in low (0.5% V/V) serum media. Fluorescently labeled polystyrene particles were added and diluted either to final concentrations of 0.0007-0.003% w/v **(****Fig. 3A****),** or fixed concentrations of 5% (v/v) otherwise, for O/N incubation in fresh full medium. Controls were done of cells without particles. The results were calculated using the equation $Normalized Uptake = \frac{{I}^{w _ beads} - {I}^{control}}{{A}^{baeds}} ⋅ Uptake _ scale$

Where *I*^{*w*_*beads*} and *I^{control}* are the fluorescence intensities of, respectively, cells after incubation with beads, and control cells without beads. In the case of FACS, *I^{w_beads}* and *I^{control}* were the median values obtained in the FACS histograms. In the case of the microplate reader they were the averages over fluorescence intensities

After the incubation with particles, the cells were washed with cold PBS, detached using trypsin, washed again and filtered through a 40-50 µm nylon mesh using a 50 ml conical tube to remove tissue debris mesh. Cells were then centrifuged and suspended in a FACS buffer containing 1% Bovine Serum Albumin in PBS and 0.05% Sodium Azide. Analyses were performed using a Beckman & Coulter CytoFlex (USA) flow cytometer and analyzed using CytExpert software.

Further quantification of particle uptake by cells was done by Fluorescence detection using microplate reader (SYNERGY-HT, Biotek, USA). The tested cells were seeded in a final concentration of 20,000 cells per well in 96 well clear bottom plates (Corning, Sigma) and cultured till cells reach 90% confluency. Polystyrene beads were added from 1% stock to a final concentration of 5% (v/v). The results were calculated using the equation above. Cells were incubated with beads for 24 h, washed thoroughly with PBS and read with microplate reader in Excitation: 530/25, Emission: 590/20. Cells without beads were used to obtain a baseline signal.

Cell imaging was used as a direct detection of particle uptake we used, both by inverted fluorescent microscope (Olympus Corporation, Japan, model IX73) and by confocal microscopy (Nikon's A1 MP multi-photon confocal microscope equipped with 639 nm diode). For Fluorescence Microscopy cells were seeded on 24 well plates. After O/N starvation (serum-free medium), fluorescently labeled beads, in the size of 0.5 µm-2.4 µm were added for an O/N incubation. The cells were then washed repeatedly and fixed using 4% paraformaldehyde (PFA) and counter-stained with 6-diamidino-2-phenylindole (DAPI). Wells were then observed and photographed using fluorescent microscopy (Olympus IX-73).

To further validate the extent of particle uptake, and differentiate between internalization and cell adhesion, confocal microscope with optical sectioning was used. Cells were seeded in Ibidi 8 well µ-plates for 3 days, during which cells were starved O/N and later incubated O/N with fluorescent labeled beads ranging from 0.05 to 2.4 µm in fresh full medium. Cells were then washed thoroughly, fixated with 4% PFA and stained with 4',6-diamidino-2-phenylindole (DAPI) for nuclei staining with or without counter-staining of Alexa Fluor *488*^{®} *phalloidin* for actin staining and. Wells were imaged and photographed using a ZEISS (Germany) confocal microscope. Z-stacks (~1 µm/slice) were performed for ortho- and 3D-analysis using ZEISS Zen software.

### Time-lapse imaging of fluorescently tagged-polystyrene particle uptake

Real time imaging of particle uptake was performed using the confocal microscope. Further details and movies are presented in SI.

### Atomic Force Microscopy (AFM)

To evaluate the elasticity modulus of the cell membrane and adhesion to the silicon tip, indentation measurements were performed using an atomic force microscope. All measurements were performed using Nanowizard^{®} 3 (JPK Instruments, Germany). Indentation measurements were performed in contact mode using silicon oxide colloidal AFM probes (6.2 µm diameter, SQube, CP-PNPL-SiO-C, NanoAndMore GMBH, Germany). Each cell was probed at 50 points in two different locations with an area of 0.5 µm² each. To measure the elasticity modulus of the cell membrane and to avoid effects caused by the cell nucleus, indentation was performed away from the nucleus with a force of 1 nN at a speed of 5 µm/sec. The data was analyzed using JPK Instrument data analysis software. The Young's modulus was determined using a Derjaguin-Muller-Toporov (DMT) model and the maximal adhesion force was calculated by the minima point of the force curve. The mechanical deformability of cells was evaluated based on their Young's modulus and maximal adhesion force. Cells that were more deformable had higher compliance to shape changes induced by indentation or retraction of the AFM probe. Higher deformability therefore corresponded to lower values of Young's modulus and higher values of the maximal adhesion force. Cells that have small values of Young's modulus are highly elastic, and can greatly deform and allow massive indentation of the probe. High values of the maximal adhesion force represent cells that showed large deformation keeping their membrane adhered to the retracting probe.

### Cell invasion assay

To assess the migratory and invasiveness potential of cells, we measured spatial cell invasion from multicellular cancer spheroids (3-dimentional culture) in extracellular matrix. Briefly, cells were seeded in a multiwall 2% Agarose micro-wells array templated with a Master 3D Petri Dish^{®} 96 well arrays at 3,000 cells/well (Microtissues Inc., USA) as previously described, 3,000 cells/well. Following cell seeding, the micro-wells were incubated with fresh medium to allow spheroid formation for 48 h. Forty-eight hours post-spheroids incubation, the spheroids were harvested and embedded in a solution of 0.25% methyl cellulose in suitable media. Spheroids were then mixed with collagen (Rat tail collagen type I, Corning ^{®}, USA) in neutral pH, seeded in a 24 well plate and incubated for 30 min at 37 °C. After gel stabilization, 300 µl of fresh media was added in order to provide sufficient media supply. The gel containing the spheroids was gently detached from the well wall and Images of the spheroids were taken by an inverted fluorescent microscope (Olympus Corporation, Japan, model IX73) every few hours.

### FACS sorting

Generation of two sub-cell populations originated from a single cell line, differing by their capacity to uptake 2.4 µm beads, was done by the FACS sorter. Each FACS sorting procedure separated cells that did not uptake any particles ("negative", FL2^{Low}) from the rest of the cells ("positive", uptake of at least one bead, FL^{high}). Therefore, after the first sorting, in each cycle we obtained four different sub-sets of cells: positive-positive; positive-negative, negative-positive, negative-negative. Only the positive-positive and negative-negative were kept for further sorting. In each cycle the sorting enrichment, Δ (presented in **Fig. 5C**), was defined as $Δ ≡ {J}_{+}^{+} - {J}_{+}^{-}$ where ${J}_{+}^{+}$ was the percent of positive cells obtained in a sorting procedure on the positive-positive population of the previous cycle, and ${J}_{+}^{-}$ was the percent of positive cells obtained in a sorting procedure on the negative-negative population of the previous cycle. An exception to this definition is the first cycle where both of the percent values of positive cells were calculated relative to the same origin population of cells, yielding ${J}_{+}^{+} - {J}_{+}^{-} = 0$. Each sorting included the flowing steps. Cells were cultured for five generations and incubated with 2.4 µm beads for 24 h. Cells were then washed thoroughly with PBS and detached with trypsin, centrifuged, counted and diluted to 1.0×10⁷ cells/ml in PBS. Using a BD-FACSAria-III cell sorter, cells were analyzed for fluorescence intensity distribution (FL-2).

### Xenograft in vivo experiment

Following the fifth sorting cycle, A375 cells were first cultivated to dispose of the sorting particles, harvested and injected (5×10⁶ cells in 100 µL PBS containing 5% matrigel) subcutaneously (S.C) to male Athymic Nude-Foxn1 HSD 5-6 weeks old mice, under isofluorene anesthesia. Mice were inspected and weighed every other day. From day 7 of the injection, tumors were measurable, and their volume calculated through the ellipsoid equation (square of the tumor width and multiplied by 0.52). Twenty-two days after the injection, mice were sacrificed by cervical dislocation under ketamine/xylazine sedation and tumors were extracted, weighed and photographed. Tumors were fixated in formalin and embedded in paraffin for histological examination.

### Histology

Tumors extracted were embedded in paraffin and sectioned to 8 µm samples. Samples were stained with a hematoxylin and eosin (H&E) dye for detecting tissue structure using a standard procedure. For immunofluorescence, sections were fixed and 0.1% Triton was used for tissue permeability. After blocking with 3% normal goat serum, sections were incubated with primary antibodies for apoptosis and proliferation (cleaved Caspase 3 and Ki67 respectively), and three antibodies for EMT: anti-SMA, anti-E-cadherin and anti-Snail/Slug. Alexa Fluor 488 or Alexa Fluor 647 (Abcam) was used for secondary antibody labeling, and fluorescent mounting media was used with DAPI (Vectashield, Vector Laboratories, USA) for imaging using an fluorescent microscope (Olympus Corporation, Japan).

### RNA extraction

For RNA extraction the standard QIAzol protocol was used. Tissue samples were homogenized in a QIAzol Lysis Reagent. After addition of chloroform, the homogenate was separated into aqueous and organic phases by centrifugation. RNA partitions to the upper, aqueous phase, while DNA partitions to the interphase and proteins to the lower, organic phase. RNA was precipitated from the aqueous phase by addition of isopropanol. The pellet was then washed with ethanol and redissolved in RNase-free water.

### RNA Sequencing

Sequencing of positive and negative cell samples was performed in G-INCPM - Weizmann Institute of Science (Israel). Libraries were prepared using the INCPM-mRNA-seq. Briefly, the polyA fraction (mRNA) was purified from 500 ng of total RNA following by fragmentation and the generation of double-stranded cDNA. Then, end repair, A base addition, adapter ligation and PCR amplification steps were performed. Libraries were evaluated by Qubit (Thermo fisher scientific) and TapeStation (Agilent). Sequencing libraries were constructed with barcodes to allow multiplexing of 12 samples in one lane. Around 21 million single-end 60-bp reads were sequenced per sample on Illumina HiSeq 2500 V4 instrument.

### Bioinformatics

Reads were trimmed using cutadapt and mapped to the human genome GRCh38 using STAR v2.4.2a (with End To End option and out Filter Mismatch NoverLmax set to 0.04). Counting proceeded over genes annotated in Ensembl release 88, using a htseq-count (intersection-strict mode). Differential expression analysis was performed using DESeq2 with the betaPrior, cooksCutoff and independent Filtering parameters set to false. Raw P values were adjusted for multiple testing using the procedure of Benjamini and Hochberg⁵³. The pipeline was constructed using Snakemake.

### Statistical analysis

All experiments were performed and repeated three or more times. A parametric test and a two-tailed Student's t-test were used for calculating significant differences between two sets of results. An ANOVA test followed by a Tukey post hoc test were used for multiple comparisons. Error bars represent standard errors. A small fraction of outliner points were discordant in the AFM data and were subtracted using a z-test with 95% confidence.

### Gene set enrichment analysis (GSEA)

Whole differential expression data were subjected to gene set enrichment analysis using GSEA. GSEA uses all differential expression data (cut-off independent) to determine whether *a priori* defined sets of genes show statistically significant, concordant differences between two biological states. Gene sets with cytoskeleton-related functions were extracted from the molecular signatures database (MSigDB, v6.1, October 2017).

### Network analysis

Protein-protein interactions data were extracted from HIPPIE and STRING, and images were generated using Cytoscape.

### Theoretical model

The free energy change in the adhesive interaction between a rigid spherical particle and a cell is presented in the equation below. The mechanism of particle engulfment is provided in a coarse-grained manner with no attempt to understand details of the cytoskeletal dynamics, which is beyond the scope of the present work. In our simplified scheme, a single particle is considered neglecting particle-particle interactions. An initial stable envelope of the bead by the cell membrane is hypothesized to be a necessary step for full engulfment and particle uptake, and therefore our model provides the upper limit of the extent of particle internalization. For this aim, a cell is assumed to behave as a continuum 3D elastic material wrapped by an elastic 2D membrane. We used the DMT model for describing the free energy of the cohesion and the 3D cell deformation, consistent with our DMT based calculations of parameters from the AFM measurements. The Helfrisch theory is used for describing membrane elasticity. The DMT model accounts for attractive interactions of adhesion and VdW that are not spatially limited to the cell-bead contact area. The interactions are assumed to be elastic, describing the case of low penetration. The bead is assumed to be small as compared to the cell and the spontaneous curvature of the cell is taken as zero so that the initial cell geometry is modelled as half a space. The bead and cell surfaces are assumed to be smooth and tightly adhering along their contact surface. Smaller factors of the free energy, such as entropic terms and membrane-stretching tension, are neglected in our coarse-grained model as well as coupling between the DMT and Helfrich terms. The main purpose was to introduce a model as simple as possible that captured the main players in the mechanical mechanism of particle uptake in the context of the TrC. In spite of the model's simplicity, excellent quantitative agreement was found between the model predictions and our experimental data. This indicates that the model components are well-suited for describing the interactions of cells with the wide size range of particles studied here. The derivation leading to the equation below. According to the DMT model, the force *x* upon particle wrapping is given by: $χ = \frac{4}{3} {ER}^{0.5} {z}^{1.5} - 2 π R Δ γ$ providing the following energetic term: ${F}_{DMT} = {\int }_{0}^{z} χ \left(z′\right) dz ′ = \frac{8}{15} {ER}^{0.5} {z}^{2.5} - 2 π R Δ γz$

The effective Young's modulus, E, in the case of a rigid bead is given by E ***=*** $\frac{{E}_{1}}{1 - {υ}_{1}^{2}}$ where *E*₁ and *v*₁ are, respectively, the elastic modulus and Poisson ratio of the cell, and z is the indentation depth.

Upon contact with a bead, the membrane geometry is approximated as a spherical cap surrounded by a torus sector rim (red and yellow lines, respectively, in Fig. 3D). In this simplified case, the bending energy can be written analytically as: ${F}_{mem} = 0.5 κ \left(\frac{{a}_{c}}{{R}^{2}}+\frac{{a}_{r}}{{{R}_{r}}^{2}}\right)$

The cap area, *a_{c},* can be expressed in terms of *ϑ* as 2*πR*² (1 - *cosϑ*) with the bead radius R being the radius of curvature. *aᵣ,* the rim surface area is: $\begin{matrix}{a}_{r} = 2 {πR}_{r} {\int }_{0}^{ϑ} \left(\left(R+{R}_{r}\right)sinϑ-{R}_{r}sinα\right) dα = \\ 2 {πR}_{r} \left(R+{R}_{r}\right) sinϑ ⋅ ϑ + 2 {{πR}_{r}}^{2} \left(cosϑ-1\right)\end{matrix}$ where *Rᵣ* is the rim radius of curvature, which in our model is the radius of the torus tube. Scaling arguments imply that *Rᵣ* ∝ *R* for the following simple reason. If we multiply the dimensions of the bead by a given factor, this is equivalent to scaling the whole cell-bead system by the same factor as long as we remain within the regime where the cell can be modeled as half a space. Thus, the rim radius of curvature should be multiplied by the same factor as well. A direct consequence is that the radius of rim curvature should be proportional to the bead size. In our simplified derivation, we assume a constant *Rᵣ* equal to the bead radius: ${R}_{r} \approx R$

Thus summing the membrane terms we obtain: ${F}_{mem} = 2 πκsinϑ ⋅ ϑ$

From Eq. [9] we obtain z = 2*R*(1 - *cosϑ*) and therefore the DMT term can be written as a function of *ϑ*, yielding the total change in free energy as written in Eq. 1 of the main text: $Δ F \left(ϑ\right) = - 2 {πR}^{2} Δ γ \left(1-cosϑ\right) + 3 {ER}^{3} {\left(1-cosϑ\right)}^{2.5} + 4 πκsinϑ ⋅ ϑ$

Under small angle approximations (*sintϑ* ≈ *ϑ* and $1 - cosϑ \approx \frac{{ϑ}^{2}}{2}$) we obtain: $Δ F \left(ϑ\right) = \left(-{πR}^{2}Δγ+4πκ\right) {ϑ}^{2} + \sqrt{\frac{9}{32}} {ER}^{3} {ϑ}^{5}$ where Δ*F*(*ϑ*) has a minimum (for *ϑ* ≠ 0) when: ${ϑ}^{˜} = \sqrt[3]{\frac{0.75 π}{ER} \left(Δγ-\frac{4 κ}{{R}^{2}}\right)}$

This provides the following condition for stable adhesion of the NPs to the cell: $\begin{matrix}R > {R}_{c} & where & {R}_{c} ≡ \sqrt{\frac{4 κ}{Δ γ}}\end{matrix}$

This is a very significant result. For small beads below *R_{c}*, Δ*F* is a monotonically increasing function and therefore it is not expected that particles would be wrapped and taken by cells, as indeed observed in our experimental results (Figs. 2A-B and **Figs. 3A-****B).** Most intuitively, *R_{c}* increases with the membrane bending modulus, *κ* (as for small beads, 3D cell deformations are less relevant and the main energetic penalty comes from membrane deformation), and decreases with the adhesion gain (in terms of Δ*γ*).

### Fingerprint patterns of uptake can indicate on the malignancy: physical model.

For a comprehensive study of the potential in mechanical targeting in cancer, we have constructed a physical model to describe particle - cell interaction. The physical model aids in predicting the potential of mechanical based specificity beyond the experimental scope. The validity of the model has been tested by massive comparison to our experimental data. The excellent agreement found proved that the model described below provides a reliable description which captures the important physical factors involved in the process of particle engulfment and uptake by cells. An initial stable envelope of the bead by the cell membrane is hypothesized to be a necessary step for full engulfment and particle uptake. As a consequence, our model predicts the upper limit of particle internalization into cells. Fortunately, we found that what was predicted to be valid only as an upper limit, predicted nicely all experimental results. An optional plan in our proposal was to further examine the system using other physical tools (e.g., simulations), to describe the out of equilibrium processes involved in the internalization of particles into cells, if needed. The good agreement of the thermodynamic model with the experiments reduced this need.

In order to study the particle - cell interaction, our model describes the free energy change in the adhesive interaction between a rigid spherical particle and a cell. The mechanism of particle engulfment is provided in a coarse-grained manner. A single particle is considered, neglecting particle-particle interactions. As noted, an initial stable envelope of the bead by the cell membrane is hypothesized to be a necessary step for full engulfment and particle uptake. A cell is assumed to behave as a continuum 3D elastic material wrapped by an elastic 2D membrane. We used the DMT model for describing the free energy of the cohesion and the 3D cell deformation, consistent with our DMT based calculations of parameters from the AFM measurements, Calculation of Young's modulus value by means of AFM. The Helfrisch theory is used for describing membrane elasticity. The DMT model accounts for attractive interactions of adhesion and VdW that are not spatially limited to the cell-bead contact area. The interactions are assumed to be elastic, describing the case of low penetration. The bead is assumed to be small compared with the cell and the spontaneous curvature of the cell is taken as zero so that the initial cell geometry is modelled as half a space. The bead and cell surfaces are assumed to be smooth and tightly adhering along their contact surface. Smaller factors of the free energy, such as entropic terms and membrane-stretching tension, are neglected in our coarse-grained model as well as coupling between the DMT and Helfrich terms. In spite of the model's simplicity, excellent quantitative agreement was found between the model predictions and our experimental data. This indicates that the model components are well-suited for describing the interactions of cells with the wide size range of particles studied here.

**Fig. 16** shows a surface plot of the change in free energy which represents the strength of Mechanical Targeting as a predictive tool for diagnostics and personalized drug design. The lower is the free energy change the higher is the uptake. The x axis represents elastic moduli of cells, which is a biomarker of malignancy. In this example reporting beads of different diameter (y axis) are used and the fingerprint uptake pattern (e.g. the cross points of the dashed lines) can indicate on the level of cell malignancy. This important mechanical based description of the malignancy for specific patients is crucial for both informative diagnostics and precision medicine.

Matrix stiffness regulates cell behavior in various biological contexts. We examined how cancer cell uptake depends on particle size and surface rigidity.

To evaluate the survival of cells on the polyacrylamide fabricated matrices of different rigidities, we determined cells viability 12 and 96 hours after seeding **(****Fig. 17****).** Also, survival of cells grown in commercial substrates for 12 hours was measured. No major changes in viability were detected, with less than 20% of average cell death in all conditions, indicating that the polyacrylamide substrates as well as commercial substrates were not toxic to the cells. It can be seen that with rigid surfaces the cell viability is higher.

The exposed cell surface that is available to particle absorption is a critical steric factor that greatly influences the extent of particle uptake. This factor was evaluated using image analysis of cells plated on the different substrates for 12 hours, which indicates on their spreading when in contact with particles **(****Fig. 18****).** There is a clear increase in the cell area in correlation with the surface rigidity. Overall, cells exhibited lower spreading and higher circularity on the softer gels than the stiffer gels.

### A physical model for the role of surface rigidity in particle uptake.

Plating cells on surfaces of different elastic moduli affect several aspects and in result can control the extent of particle uptake by the cells. Our results indicate on an increase in uptake followed by a decrease and an additional increase - when the surface rigidity raises. The purpose of the present model is to provide insight on the physical terms that can contribute to this interesting dependence.

The model presented here is based on combination of contact mechanics and Helfrisch model. The basic derivation, for the case of an infinitely rigid surface was detailed at Brill-Karniely et al., Sci Adv. According to the derivation outlined in this work, the DMT term of the change in free energy upon particle engulfment is given by ${F}_{DMT} = {\int }_{0}^{z} χ \left(z′\right) dz ′ = \frac{8}{15} {ER}^{0.5} {z}^{2.5} - 2 π R Δ γz$

This term describes the three-dimensional interaction of the particle and the cells. The effective Young's modulus, E, in the case of a rigid bead is given by $E = \frac{{E}_{1}}{1 - {υ}_{1}^{2}}$ where *E*₁ and *v*₁ are, respectively, the elastic modulus and Poisson ratio of the cell, and z is the indentation depth.

Upon contact with a bead, the membrane geometry is approximated as a spherical cap surrounded by a torus sector rim.

This provides the change in bending energy ${F}_{mem} = 0.5 κ \left(\frac{{a}_{c}}{{R}^{2}}+\frac{{a}_{r}}{{{R}_{r}}^{2}}\right)$, where the cap area, *a_{c},* can be expressed in terms of *ϑ* as 2*πR*²(1 - *cosϑ*) with the bead radius *R* being the radius of curvature. *aᵣ,* the rim surface area is: *aᵣ =* 2*πRᵣ*(*R* + *Rᵣ*)*sinϑ* • *ϑ* + 2*πRᵣ*²(*cosϑ* - 1)
where *Rᵣ* is the rim radius of curvature (the radius of the torus tube). Scaling arguments imply that *Rᵣ* ∝ *R* for the following simple reason. If we multiply the dimensions of the bead by a given factor, this is equivalent to scaling the whole cell-bead system by the same factor as long as we remain within the regime where the cell can be modeled as half a space. Thus, the rim radius of curvature should be multiplied by the same factor as well. A direct consequence is that the radius of rim curvature should be proportional to the bead size. In our simplified derivation, we assume a constant *Rᵣ* equal to the bead radius: *Rᵣ* ≈ *R.* Thus, summing the membrane terms yields *Fₘₑₘ* = 2*πκsinϑ* ▪ *ϑ*

Since z = 2R(1 - *cosϑ*) DMT term can be written as a function of *ϑ*, yielding the total change in free energy, Δ*F*(*ϑ*) = -2*πR*²*Δγ*(1 - *cosϑ*) + 3*ER*³ (1 - *cosϑ*)^{2.5} + 4*πκsinϑ* ▪ *ϑ*

In our simplified scheme we use small angle approximations providing the free energy change in the process of particle engulfment by cells, for the case of completely rigid surfaces: $Δ {F}^{ξ \to ∞} \left(ϑ\right) = \left(-{πR}^{2}Δγ+4πκ\right) {ϑ}^{2} + \sqrt{\frac{9}{32}} {ER}^{3} {ϑ}^{5}$

Where S, is Young's modulus of the surface.

Minimizing this expression provides the stable (semi-equilibrium) angle of bead - cell attachment for a rigid surface: ${{ϑ}^{˜}}^{ξ \to ∞} = \sqrt[3]{\frac{0.75 π}{ER} \left(Δγ-\frac{4 κ}{{R}^{2}}\right)}$

The effect of ξ is inserted to this model in several level. First, it affects the Young's modulus of the cell, E(ξ). It also controls cell spreading area, A(ξ). Additional effects are on cell viability and on the size of the stable contact area, which provides an additional steric factor. Based on the literature listed below we use a power law fit for Young's modulus and the cell area as follows: $\begin{matrix}E \left(ξ\right) = {aξ}^{b} & and & A \left(ξ\right) = {cξ}^{d}\end{matrix}$

A rough fit of the literature data was done here based on Jamney paper ("fit parameters based on literature" excel file), see below:
*E*(*ξ*) = 0.8*ξ*^{0.27} where E and ksi are both in kPA units and *A*(*ξ*) = 700*ξ*^{0,3} where A is in um^2 units.

In our model we use E in units of kT/pm3. Typical values of E for According to Jamney's work, a typical value for a rigid substrate is 2 kPa.

Thus, we use values of a around 0.8*30000=24000 kT/µm^3

Thus: *E*(*ξ*) = 24000*ξ*^{0.27} in kT/µm^3 and *A*(*ξ*) = 700*ξ*^{0.3} in um^2 are used as estimated in our model. ${ϑ}^{˜} \left(ξ\right) = \sqrt[3]{\frac{0.75 π}{{aξ}^{b} R} \left(Δγ-\frac{4 κ}{{R}^{2}}\right)}$

And the free energy change in the equilibrium as a function of ksi: $\begin{matrix}{Δ F}^{˜} \left(R, ξ\right) = \left(-{πR}^{2}Δγ+4πκ\right) {\left[\frac{0.75 π}{{aξ}^{b} R}\left(Δγ-\frac{4 κ}{{R}^{2}}\right)\right]}^{2 / 3} \\ + \sqrt{\frac{9}{32}} {aξ}^{b} {R}^{3} {\left[\frac{0.75 π}{{aξ}^{b} R}\left(Δγ−\frac{4 κ}{{R}^{2}}\right)\right]}^{5 / 3}\end{matrix}$

The uptake probability is approximated with Boltzman factor including the steric effect of the area dependence on ksi. Here we do not consider the effect of cell viability (w/o v), nor the steric factor of the contact area (w/o a_{c}): ${P}^{w / o v , ac} \left(R, ξ\right) ∝ {cξ}^{d} {e}^{- {Δ F}^{˜} \left(R, ξ\right)}$

In our experiments a non-monotonic dependence was found with an initial increase, then a decrease and another increase. When ignoring the viability and contact steric area terms this trend was not found in the model, as shown below.

An increasing function was found here.

It is reasonable to assume that the size of the stable contact area provides an additional factor that controls the rate of uptake. Namely, not only the free energy of the stable configuration controls the tendency of full engulfment, but also the size of the contact area in the stable configuration.

Adding a linear term of the contact area as a steric factor in the uptake probability expression we get: ${P}^{w / o v , w ac} \left(R, ξ\right) ∝ 2 {πR}^{2} \left(1-cos{ϑ}^{˜}\left(ξ\right)\right) {cξ}^{d} {e}^{- {Δ F}^{˜} \left(R, ξ\right)}$

We see here a sharp increase followed by an increase. We do not see the added increase that followed in the experiments. So far there were no free parameters in the model, all were taken based on our data/literature:

| ksi kPa | Rum | a kT/µm^3 | b | c um^2 | d | del_gama kT/um |
|---|---|---|---|---|---|---|
| 0.2 | 2.4 | 24000 | 0.3 | 700 | 0.3 | 6 |

An initial assumption, using a power law for the viability, provides a bi-extramum graph similar to the experimental data. Here the viability, v, is given by *v*(ξ) = (*lξ*)*^{s}* + g with l=0.0015, s=1.8 and b=0.8. These were chosen as an estimated fit to Katya's home made surfaces results. Indeed, the bi-extramum shape is observed.

We studied the uptake of inert fluorescent polystyrene particles ranging from hundredths to several microns in size by pancreatic cell lines cultured on fabricated matrices of different rigidities in vitro using imaging flow cytometer (Fig. 19). Cells were cultured on matrices of different rigidities for 12 hours. Then, 0.8 µm purple fluorescent polystyrene beads were added (incubation for 24 hours). We found that there is a non-monotonic dependence between cell uptake capacity and surface rigidity: cells that were grown on surfaces of a low rigidity internalized less beads than cells grown on surfaces of extreme rigidity, while cells on surfaces with medium rigidity internalized the highest number of beads.

Next, we expanded the range of surfaces' stiffness and used commercial matrices to examine how cell uptake capacity will be affected. We found that the increase in surface rigidity does not cause monotonic increase in cell uptake capacity, but rather there are alternating results.

In addition, we evaluated the effect of the beads' size on cell uptake capacity by comparing uptake of 0.8 vs 2.4 µm beads. Cells internalize larger beads with less efficiency, so the uptake decreases. However, the trend of non-monotonic dependence of cell uptake capacity on surface rigidity was preserved: with larger beads differences between cell uptake capacities on different surface rigidities become more apparent.

Plating cells on surfaces of different elastic moduli affect several aspects and in result can control the extent of particle uptake by the cells. Our results indicate on an increase in uptake followed by a decrease and an additional increase - when the surface rigidity raises. We believe that there are three main effects that control the cell's uptake capacity: available cell surface, cell viability and cell deformability. The available cell area for particle absorption is larger when the surface is more rigid. In addition, with rigid surfaces the cell viability is higher. These two factors support the increase of particle uptake with substrate rigidity. On the other hand, it is well known in the literature that cells are less deformable on rigid substrates. Taken together, these effects may explain the trend of particle uptake observed in our results - a non-monotonous dependence between the surface rigidity and the cell uptake capacity.

## Claims

1. A method for determining at least one cancer-related parameter in a tissue or cells sample obtained from a subject, the method comprising determining engulfment/uptake of particles by cells in said sample having been treated or contacted with a particle population comprising at least two different particle sub-populations, each of said at least two particle sub-population being distinguishable from the other by a different physical parameter and a different identifiable barcode,
wherein engulfment/uptake of at least a portion of said particle population, or a preferential engulfment/uptake of at least a portion of a sub-population, or a preferential engulfment/uptake of a combination of sub-populations determines the at least one cancer-related parameter.

2. The method according to claim 1, for determining presence of a cancer cell in the tissue or cells sample, the method comprising determining uptake or engulfment of the particles by cells in said sample having been treated or contacted with the particle population,
wherein cells engulfing/uptaking at least a portion of said particle population, or cells demonstrating preferential uptake of a particle sub-population, or cells demonstrating a preferential engulfment/uptake of a combination of sub-populations are cancer cells, provided that the cells are not macrophages, astrocytes, dendritic cells or osteoclasts.

3. The method according to claim 1, for determining presence of a cancer cell in the tissue or cell sample, the method comprising
- providing a tissue sample or a cell sample having been contacted with a particle population under conditions enabling particles uptake by cells in said sample, the particle population comprising at least two different particle sub-populations, each of said at least two particle sub-population comprises or consists particles of a different physical parameter and a different identifiable barcode; and
- determining uptake of the particles by the cells, wherein cells engulfing at least a portion of said particle population, or cells demonstrating preferential uptake of a particle sub-population, or cells demonstrating a preferential engulfment/uptake of a combination of sub-populations are cancer cells, provided that the cells are not macrophages, astrocytes, dendritic cells or osteoclasts.

4. The method according to claim 1, for determining presence of a cancer cell in a tissue or cell sample, the method comprising
- treating or contacting the tissue or cells sample *ex vivo* or *in vitro* with a particle population under conditions enabling particles uptake by cells in said sample, the particle population comprising at least two different particle sub-populations, each of said at least two particle sub-population comprises or consists particles of a different physical parameter and a different identifiable barcode; and
- determining uptake of the particles by the cells, wherein cells engulfing at least a portion of said particle population, or cells demonstrating preferential uptake of a particle sub-population, or cells demonstrating a preferential engulfment/uptake of a combination of sub-populations are cancer cells, provided that the cells are not macrophages, astrocytes, dendritic cells or osteoclasts.

5. The method according to any one of the preceding claims, wherein the cancer-related parameter is one or more of (a) presence of cancer cells in the sample, (b) grade and severity of a cancer in a sample identified as cancerous or in a subject diagnosed with cancer, (c) metastasis or invasiveness potential of a cancer in a sample identified as cancerous or in a subject diagnosed with cancer, (d) whether a particular treatment is effective as an anticancer treatment, (e) whether a potential anticancer treatment is effective against a particular cancer in a subject, and (f) whether a particular mode of delivery of a therapeutic agent for a specific cancer or patient is effective.

6. The method according to any one of the preceding claims, wherein each of the sub-populations is distinguishable by at least one identifiable barcode in a form of a tracer moiety enabling selective tracing and identification of the sub-populations.

7. The method according to claim 6, wherein the barcode is provided in a form of a chemical moiety on a surface region of the particles, or in a form of a material comprised within the particles.

8. The method according to claim 7, wherein the chemical moiety having a characteristic distinguishable signal.

9. The method according to claim 8, wherein the chemical moiety is in a form of a luminescent tag, a photoluminescent tag, a chemiluminescent tag, a fluorescent tag, a magnetic tag, an MRI tag, a colorimetric tag, a chemical tag, a hybridization tag, a polynucleotide tag, a peptide tag, a semiconductor tag, an XRF tag, an X-ray tag or a quantum dot.

10. The method according to claim 9, wherein the chemical moiety is a fluorescent tag.

11. The method according to claim 10, wherein the fluorescent tag is selected from hydroxycoumarin, aminocoumarin, methoxycoumarin, cascade blue, pacific blue, pacific orange, lucifer yellow, NBD, R-Phycoerythrin, fluorescein, G-Dye100, G-Dye200, G-Dye300, G-Dye400, Cy2, Cy3, Cy3B, Cy3.5, Cy5, Cy5.5, Cy7, Rhodamine, Lissamine Rhodamine, Texas Red, and allophycocyanin.

12. The method according to claim 11, wherein the fluorescent tag is Cy5 or Cy3.

13. The method according to any one of claims 1 to 12, comprising
- determining uptake or engulfment of particles by cells in a tissue or cell sample having been treated or contacted with a particle population comprising at least two different particle sub-populations, each of said at least two particle sub-population comprises or consists particles having or **characterized by** a different physical parameter and a different identifiable barcode,
- determining an uptake profile relating to the particle population or plurality of sub-populations and/or the amount or number of particles taken up by the cells;
- comparing said uptake profile to a fingerprint profile obtained for heathy cells utilizing a particle population identical to the particle population with which the sample was contacted;
wherein a deviation from the fingerprint profile is indicative of one or more of (i) presence of a cancer cell in the sample, (ii) absence of a cancer cell in the sample, and (iii) metastatic potential or malignancy or aggressiveness of cells in the sample.

14. The method according to claim 13, the method comprising providing a tissue sample or a cell sample having been contacted with a particle population under conditions enabling particles uptake by cells in the sample, the particle population comprising at least two different particle sub-populations, each of said at least two particle sub-population comprises or consists particles of a different physical parameter and a different identifiable barcode.

15. The method according to claim 13, the method comprising treating or contacting the tissue or cells sample *ex vivo* or *in vitro* with a particle population

## Patentansprüche

1. Verfahren zur Bestimmung wenigstens eines krebsbezogenen Parameters in einer Gewebe- oder Zellprobe, die von einem Subjekt erhalten wurde, wobei das Verfahren die Bestimmung den Einschluss/die Aufnahme von Partikeln durch Zellen in der Probe umfasst, die mit einer Partikelpopulation behandelt oder in Kontakt gebracht wurden, die wenigstens zwei unterschiedliche Partikel-Subpopulationen umfasst, wobei jede der wenigstens zwei Partikel -Subpopulationen von der anderen durch einen unterschiedlichen physikalischen Parameter und einen unterschiedlichen identifizierbaren Strichcode unterscheidbar ist,
wobei der Einschluss/das Aufnehmen von wenigstens einem Teil der Partikelpopulation oder ein bevorzugtes Einschließen/Aufnehmen von wenigstens einem Teil einer Subpopulation oder ein bevorzugtes Einschließen/Aufnehmen einer Kombination von Subpopulationen den wenigstens einen krebsbezogenen Parameter determiniert.

2. Verfahren nach Anspruch 1, zur Bestimmung des Vorhandenseins einer Krebszelle in der Gewebe- oder Zellprobe, wobei das Verfahren die Bestimmung der Aufnahme oder des Einschlusses der Partikel durch Zellen in der Probe umfasst, die mit der Partikelpopulation behandelt oder in Kontakt gebracht wurden,
wobei die Zellen, die wenigstens einen Teil der Partikelpopulation einschließen/aufnehmen, oder die eine bevorzugte Aufnahme einer Partikel-Subpopulation zeigen, oder die ein bevorzugtes Einschließen/Aufnehmen einer Kombination von Subpopulationen zeigen, Krebszellen sind, mit der Maßgabe, dass die Zellen keine Makrophagen, Astrozyten, dendritische Zellen oder Osteoklasten sind.

3. Verfahren nach Anspruch 1, zur Bestimmung des Vorhandenseins einer Krebszelle in der Gewebe- oder Zellprobe, wobei das Verfahren umfasst:
- Bereitstellen einer Gewebeprobe oder einer Zellprobe, die mit einer Partikelpopulation unter Bedingungen in Kontakt gebracht wurde, die die Aufnahme von Partikeln durch Zellen in der Probe ermöglichen, wobei die Partikelpopulation wenigstens zwei unterschiedliche Partikel-Subpopulationen umfasst, wobei jede der wenigstens zwei Partikel-Subpopulationen Partikel mit einem unterschiedlichen physikalischen Parameter und einem unterschiedlichen identifizierbaren Strichcode umfasst oder daraus besteht; und
- Bestimmen der Aufnahme der Partikel durch die Zellen, wobei Zellen, die wenigstens einen Teil der Partikelpopulation einschließen, oder Zellen, die eine bevorzugte Aufnahme einer Partikel-Subpopulation zeigen, oder Zellen, die ein bevorzugtes Einschließen/Aufnehmen einer Kombination von Subpopulationen zeigen, Krebszellen sind, mit der Maßgabe, dass die Zellen keine Makrophagen, Astrozyten, dendritische Zellen oder Osteoklasten sind.

4. Verfahren nach Anspruch 1, , zur Bestimmung des Vorhandenseins einer Krebszelle in der Gewebe- oder Zellprobe, wobei das Verfahren umfasst:
- Behandeln oder Kontaktieren der Gewebe- oder Zellprobe *ex vivo* oder *in vitro* mit einer Partikelpopulation unter Bedingungen, die die Aufnahme von Partikeln durch Zellen in der Probe ermöglichen, wobei die Partikelpopulation wenigstens zwei unterschiedliche Partikel-Subpopulationen umfasst, wobei jede der wenigstens zwei Partikel-Subpopulationen Partikel mit einem unterschiedlichen physikalischen Parameter und einem unterschiedlichen identifizierbaren Strichcode umfasst oder daraus besteht; und
- Bestimmen der Aufnahme der Partikel durch die Zellen, wobei Zellen, die wenigstens einen Teil der Partikelpopulation einschließen, oder Zellen, die eine bevorzugte Aufnahme einer Partikel-Subpopulation zeigen, oder Zellen, die ein bevorzugtes Einschließen/Aufnehmen einer Kombination von Subpopulationen zeigen, Krebszellen sind, mit der Maßgabe, dass die Zellen keine Makrophagen, Astrozyten, dendritische Zellen oder Osteoklasten sind.

5. Verfahren nach einem der vorhergehenden Ansprüche, wobei es sich bei dem krebsbezogenen Parameter um einen oder mehrere der folgenden Parameter handelt: (a) Vorhandensein von Krebszellen in der Probe, (b) Grad und Schweregrad eine Krebses in einer als krebsentartet identifizierten Probe oder in einem mit Krebs diagnostizierten Subjekt, (c) Metastasierungs- oder Invasionspotenzial eines Krebses in einer als krebsentartet identifizierten Probe oder in einem mit Krebs diagnostizierten Subjekt, (d) ob eine bestimmte Behandlung als Anti-Krebs-Behandlung wirksam ist, (e) ob eine potenzielle Anti-Krebs-Behandlung gegen einen bestimmten Krebs bei einem Subjekt wirksam ist, und (f) ob eine bestimmte Art der Verabreichung eines therapeutischen Agens für einen bestimmten Krebs oder Patienten wirksam ist.

6. Verfahren nach einem der vorhergehenden Ansprüche, wobei jede der Subpopulationen durch wenigstens einen identifizierbaren Strichcode in Form einer Tracer-Moiety unterscheidbar ist, die eine selektive Verfolgung und Identifizierung der Subpopulationen ermöglicht.

7. Verfahren nach Anspruch 6, wobei der Strichcode in Form einer chemischen Moiety auf einem Oberflächenbereich der Partikel oder in Form eines Materials in den Partikeln bereitgestellt wird.

8. Verfahren nach Anspruch 7, wobei die chemische Moiety ein charakteristisches unterscheidbares Signal aufweist.

9. Verfahren nach Anspruch 8, wobei die chemische Moiety in Form eines Lumineszenz-Tags, eines Photolumineszenz-Tags, eines Chemilumineszenz-Tags, eines Fluoreszenz-Tags, eines magnetischen Tags, eines MRI-Tags, eines kolorimetrischen Tags, eines chemischen Tags, eines Hybridisierungs-Tags, eines Polynukleotid-Tags, eines Peptid-Tags, eines Halbleiter-Tags, eines XRF-Tags, eines Röntgen-Tags oder eines Quantenpunkts gebildet ist.

10. Verfahren nach Anspruch 9, wobei die chemische Moiety ein Fluoreszenz-Tag ist.

11. Verfahren nach Anspruch 10, wobei der Fluoreszenz-Tag ausgewählt ist aus Hydroxycumarin, Aminocumarin, Methoxycumarin, Kaskadenblau, Pazifikblau, Pazifikorange, Luzifergelb, NBD, R-Phycoerythrin, Fluorescein, G-Dye100, G- Dye200, G- Dye300, G-Dye400, Cy2, Cy3, Cy3B, Cy3.5, Cy5, Cy5.5, Cy7, Rhodamin, Lissamin Rhodamin, Texas Red und Allophycocyanin.

12. Verfahren nach Anspruch 11, wobei der Fluoreszenz-Tag Cy5 oder Cy3 ist.

13. Verfahren nach einem der Ansprüche 1 bis 12, umfassend:
- Bestimmen der Aufnahme oder des Einschließens von Partikeln durch Zellen in einer Gewebe- oder Zellprobe, die mit einer Partikelpopulation behandelt oder in Kontakt gebracht wurde, die wenigstens zwei unterschiedliche Partikel-Subpopulationen umfasst, wobei jede der wenigstens zwei Partikel -Subpopulationen Partikel umfasst oder aus daraus besteht, die einen unterschiedlichen physikalischen Parameter und einen unterschiedlichen identifizierbaren Strichcode aufweisen oder **dadurch gekennzeichnet** sind,
- Bestimmen eines Aufnahmeprofils in Bezug auf die Partikelpopulation oder Mehrzahl von Partikel-Subpopulationen und/oder die Menge oder Anzahl von durch die Zellen aufgenommenen Partikel;
- Vergleichen des Aufnahmeprofils mit einem Fingerprint-Profil, das für gesunde Zellen unter Verwendung einer Partikelpopulation erhalten wurde, die mit der Partikelpopulation identisch ist, mit der die Probe in Kontakt gebracht wurde;
wobei eine Abweichung von dem Fingerprint-Profil indikativ ist für (i) das Vorhandensein einer Krebszelle in der Probe, (ii) das Fehlen einer Krebszelle in der Probe, und/oder (iii) das Metastasierungspotenzial oder die Bösartigkeit oder Aggressivität von Zellen in der Probe.

14. Verfahren nach Anspruch 13, wobei das Verfahren die Bereitstellung einer Gewebe- oder Zellprobe umfasst, die mit einer Partikelpopulation unter Bedingungen, die die Aufnahme von Partikeln durch die Zellen in der Probe ermöglichen, wobei die Partikelpopulation wenigstens zwei unterschiedliche Partikel-Subpopulationen umfasst, wobei jede der wenigstens zwei Partikel-Subpopulationen Partikel mit einem unterschiedlichen physikalischen Parameter und einem unterschiedlichen identifizierbaren Strichcode umfasst oder daraus besteht.

15. Verfahren nach Anspruch 13, wobei das Verfahren die Behandlung oder das Inkontaktbringen der Gewebe- oder Zellenprobe *ex vivo* oder *in vitro* mit einer Partikelpopulation umfasst.

## Revendications

1. Méthode en vue de la détermination d'au moins un paramètre en relation avec le cancer dans un tissu ou un échantillon cellulaire obtenu à partir d'un sujet, la méthode comprenant la détermination d'un engloutissement/d'une absorption de particules par des cellules dans ledit échantillon ayant été traité ou mis en contact avec une population de particules comprenant au moins deux sous-populations de particules différentes, chacune desdites au moins deux sous-populations de particules étant distinctibles l'une de l'autre par un paramètre physique différent et un code à barres différent et identifiable,
dans laquelle l'engloutissement/absorption d'au moins une partie de ladite population de particules, ou un engloutissement/une absorption de préférence d'au moins une partie d'une sous-population, ou un engloutissement/une absorption de préférence d'une combinaison de sous-populations détermine au moins l'un des paramètres liés au cancer.

2. Méthode selon la revendication 1 en vue de la détermination de la présence d'une cellule cancéreuse dans le tissu ou l'échantillon cellulaire, la méthode comprenant la détermination de l'absorption ou de l'engloutissement des particules par les cellules dans ledit échantillon ayant été traité ou mis en contact avec la population de particules,
dans laquelle les cellules engloutissant/absorbant au moins une partie de ladite population de particules, ou les cellules manifestant une absorption de préférence d'une sous-population de particules, ou les cellules manifestant un engloutissement/une absorption de préférence d'une combinaison de sous-populations sont des cellules cancéreuses, sous réserve que les cellules ne soient pas des macrophages, des astrocytes, des cellules dendritiques ou des ostéoclastes.

3. Méthode selon la revendication 1 en vue de la détermination de la présence d'une cellule cancéreuse dans le tissu ou l'échantillon cellulaire, la méthode comprenant
- la livraison d'un échantillon de tissu ou d'un échantillon cellulaire ayant été mis en contact avec une population de particules dans des conditions permettant l'absorption de particules par des cellules dans ledit échantillon, la population de particules comprenant au moins deux sous-populations de particules différentes, chacune desdites au moins deux sous-populations de particules comprenant ou étant constituée de particules d'un paramètre physique différent et d'un code à barres identifiable différent; et
- la détermination de l'absorption des particules par les cellules, dans laquelle des cellules engloutissant au moins une partie de ladite population de particules, ou des cellules manifestant une absorption de préférence d'une sous-population de particules, ou des cellules manifestant un engloutissement/une absorption de préférence d'une combinaison de sous-populations sont des cellules cancéreuses, sous réserve que les cellules ne soient pas des macrophages, des astrocytes, des cellules dendritiques ou des ostéoclastes.

4. Méthode selon la revendication 1 en vue de la détermination de la présence d'une cellule cancéreuse dans un tissu ou un échantillon cellulaire, la méthode comprenant
- le traitement ou la mise en contact du tissu ou de l'échantillon cellulaire ex vivo ou in vitro avec une population de particules dans des conditions permettant l'absorption des particules par les cellules dudit échantillon, la population de particules comprenant au moins deux sous-populations de particules différentes, chacune desdites au moins deux sous-populations de particules comprenant ou se composant de particules d'un paramètre physique différent et d'un code à barres identifiable différent ; et
- la détermination de l'absorption des particules par les cellules, dans laquelle des cellules engloutissant au moins une partie de ladite population de particules, ou des cellules manifestant une absorption de préférence d'une sous-population de particules, ou des cellules manifestant un engloutissement/une absorption de préférence d'une combinaison de sous-populations sont des cellules cancéreuses, sous réserve que les cellules ne soient pas des macrophages, des astrocytes, des cellules dendritiques ou des ostéoclastes.

5. Méthode selon une quelconque des revendications précédentes, dans laquelle le paramètre en relation avec le cancer est un ou plusieurs des éléments suivants : (a) présence de cellules cancéreuses dans l'échantillon, (b) degré et sévérité d'un cancer dans un échantillon identifié comme cancéreux ou chez un sujet diagnostiqué d'un cancer, (c) potentiel de métastases ou d'invasivité d'un cancer dans un échantillon identifié comme cancéreux ou chez un sujet diagnostiqué d'un cancer, (d) si un traitement particulier est efficace en tant que traitement anticancéreux, (e) si un traitement anticancéreux potentiel est efficace contre un cancer particulier chez un sujet, et (f) si un mode particulier d'administration d'un agent thérapeutique est efficace contre un cancer spécifique ou chez un patient.

6. Méthode selon une quelconque des revendications précédentes, dans laquelle chacune des sous-populations est distinctible par au moins un code à barres identifiable sous une forme d'une fraction traceuse permettant le traçage et l'identification sélectifs des sous-populations.

7. Méthode selon la revendication 6, dans laquelle le code à barres est fourni sous la forme d'une fraction chimique sur une région de surface des particules, ou sous la forme d'une matière comprise dans les particules.

8. Méthode selon la revendication 7, dans laquelle la fraction chimique possède un signal distinctible caractéristique.

9. Méthode selon la revendication 8, dans laquelle la fraction chimique se présente sous la forme d'une balise luminescente, d'une balise photoluminescente, d'une balise chimioluminescente, d'une balise fluorescente, d'une balise magnétique, d'une balise IRM, d'une balise colorimétrique, d'une balise chimique, d'une balise d'hybridation, d'une balise polynucléotidique, d'une balise peptidique, d'une balise semi-conductrice, d'une balise XRF, d'une balise à rayons X ou d'un point quantique.

10. Méthode selon la revendication 9, dans laquelle la fraction chimique est une balise fluorescente.

11. Méthode selon la revendication 10, dans laquelle la balise fluorescente est sélectionnée à partir de l'hydroxycoumarine, de l'aminocoumarine, de la méthoxycoumarine, du bleu cascade, du bleu pacifique, de l'orange pacifique, du jaune lucifer, du NBD, de la R-phycoérythrine, de la fluorescéine, du G-Dye100, du G- Dye200, du G-Dye300, du G-Dye400, du Cy2, du Cy3, du Cy3B, du Cy3,5, du Cy5, du Cy5,5, duCy7, de la rhodamine, de la lissamine rhodamine, du Texas Red et de l'allophycocyanine.

12. Méthode selon la revendication 10, dans laquelle la balise fluorescente est du Cy5 ou du Cy3.

13. Méthode selon une quelconque des revendications 1 à 12, comprenant
la détermination de l'absorption ou de l'engloutissement de particules par des cellules d'un tissu ou d'un échantillon cellulaire ayant été traité ou mis en contact avec une population de particules comprenant au moins deux sous-populations de particules différentes, chacune de ces au moins deux sous-populations de particules comprenant ou étant constituée de particules possédant ou **caractérisées par** un paramètre physique différent et un code à barres identifiable différent,
- la détermination d'un profil d'absorption relatif à la population de particules ou à la pluralité de sous-populations et/ou à la quantité ou au nombre de particules absorbées par les cellules;
- la comparaison dudit profil d'absorption avec un profil d'empreinte digitale obtenu pour des cellules saines en utilisant une population de particules identique à la population de particules avec laquelle l'échantillon a été mis en contact ;
dans laquelle une déviation par rapport au profil d'empreinte digitale est indicatrice d'un ou plusieurs des éléments suivants : (i) présence d'une cellule cancéreuse dans l'échantillon, (ii) absence d'une cellule cancéreuse dans l'échantillon, et (iii) potentiel métastatique, malignité ou agressivité des cellules dans l'échantillon.

14. Méthode selon la revendication 13, la méthode comprenant la livraison d'un échantillon de tissu ou d'un échantillon cellulaire ayant été mis en contact avec une population de particules dans des conditions permettant l'absorption des particules par des cellules dans l'échantillon, la population de particules comprenant au moins deux sous-populations de particules différentes, chacune desdites au moins deux sous-populations de particules comprenant ou étant constituée de particules d'un paramètre physique différent et d'un code à barres identifiable différent.

15. Méthode selon la revendication 13, la méthode comprenant le traitement ou la mise en contact du tissu ou de l'échantillon cellulaire ex vivo ou in vitro avec une population de particules.
